# EUROPEAN PATENT SPECIFICATION

(11) **EP 3 249 382 B1**
(45) Date of publication and mention of the grant of the patent: **02.03.2022**
(21) Application number: 16740221.3
(22) Date of filing: 20.01.2016
(51) Int. Cl.: G01N 1/30, G01N 33/48, G02B 21/00

(54) **BIOLOGICAL-SPECIMEN TRANSPARENTIZING AGENT, SYSTEM, AND USE THEREFOR**
MITTEL ZUR TRANSPARENTISIERUNG BIOLOGISCHER PROBEN, SYSTEM UND VERWENDUNG DAVON
AGENT DE TRANSPARENCE D'ÉPROUVETTES BIOLOGIQUES, SYSTÈME, ET UTILISATION ASSOCIÉE

(30) Priority: 20.01.2015 JP 2015008928
(43) Date of publication of application: 29.11.2017
(73) Proprietor: Riken, Wako-shi, Saitama 351-0198 (JP)
(72) Inventor: MIYAWAKI, Atsushi, Wako-shi Saitama 351-0198 (JP); HAMA, Hiroshi, Wako-shi Saitama 351-0198 (JP)
(74) Representative: Bittner, Thomas L.
(86) International application number: PCT/JP2016/051599
(87) International publication number: WO 2016/117614

(56) References cited:
- WO-A1-94/00986
- WO-A1-2011/111876
- WO-A1-2012/147965
- WO-A1-2014/010633
- US-A- 4 748 189
- US-A1- 2014 178 927
- M UEDA ET AL: "Effect of sorbitol and inositol on the critical micelle concentration of nonionic surfactants in water and in aqueous urea", COLLOID & POLYMER SCIENCE, vol. 257, no. 9, September 1979 (1979-09), pages 973-976, XP055508244, ISSN: 0303-402X, DOI: 10.1007/bf01520723
- TIWARI A ET AL: "Stabilization of yeast hexokinase A by polyol osmolytes: Correlation with the physicochemical properties of aqueous solutions", BIOPHYSICAL CHEMISTRY, ELSEVIER, AMSTERDAM, NL, vol. 124, no. 2, 20 November 2006 (2006-11-20), pages 90-99, XP027879403, ISSN: 0301-4622

## Description

The present invention relates to a clearing reagent for making a biological material transparent, and use thereof.

### Background

For an internal observation of a deep part of a biological material with use of an optical microscope, it is necessary to perform a treatment (a clearing treatment for making a subject transparent) with use of a clearing reagent.

Typical examples of known clearing reagents and known clearing treatment methods encompass (i) a Focus Clear (product name) solution described by Ann-Shyn Chiang in Patent Literature 1 and Non-Patent Literature 1 and (ii) a tissue clearing method described by Hans-Ulrich Dodt et al. in Non-Patent Literature 2. These are both used to make a tissue transparent for an observation of a structure which exists in the tissue and which is labeled by a fluorescent substance.

These methods require an organic solvent as an active component in a clearing treatment, and are therefore difficult to use for biological materials. In addition, these methods also have such a risk of causing shrinkage of a biological material during the clearing treatment.

In view of these problems, a clearing technique (Scale), in which a water-soluble reagent containing urea is used, has been developed. Scale established a foundation of techniques for three-dimensionally observing a structure of a biological material by making the biological material transparent. (Patent Literature 2 and Non-Patent Literature 3).

For example, after disclosure of Scale, techniques (SeeDB) for making a biological material transparent by adjusting a refractive index with use of a solution containing fructose was disclosed (Patent Literature 3 and Non-Patent Literature 4). The SeeDB method is intended to minimize denaturation of a biological material during the treatment. The results of optical microscopic observation revealed that shapes of biological materials, such as membrane structures and axons, are preserved. However, a refractive index of the observation target becomes extremely high. Consequently, for sufficient observation, the following are required: (i) a two-photon microscope and (ii) lenses optimized for high refractive index.

Non Patent Literature 5 discloses a clearing technique (CLARITY) based on a physicochemical method using an electrophoresis apparatus. CLARITY uses an electrophoresis apparatus to physicochemically treat acrylamide polymer-embedded brain tissue, for removing lipid components are removed. The brain tissue is made transparent in 2 weeks. However, CLARITY unfortunately requires a dedicated device and involves a complicated process. In addition, since electrophoresis generates heat as time elapses, it is difficult to prevent thermal denaturation of protein in a biological material.

In addition, CLARITY performs clearing by removing light scattering of lipid membrane through causing lipid to be eluted from tissue with use of ionic surfactant sodium dodecyl sulfate (SDS) at a high concentration (4%). This causes large loss, for example, of (i) structures of plasma membranes of cells and structures of secretion vesicles and (ii) proteins floating in lipid membranes.

Various techniques for clearing biological materials have been thus developed after the disclosure of the clearing technique, Scale, using urea. However, the advantages presented only by Scale are evident.

For example, Scale is free of (i) such a problem of SeeDB that non-versatile apparatuses, such as lenses optimized for high refractive index and a two-photon microscope, are necessary and (ii) such problems of CLARITY as thermal denaturation of a biological material and necessity of a dedicated device. It should be noted that although conventional Scale possess such an advantage as an extremely simple process, there is a demand for a further rapid increase in transparency (light-transmitting property) while an original form of a biological material is preserved.

Recently, a technique (CUBIC) has been developed, which is based on clarification with use of urea as with the Scale technique and which achieves excellent clarification by treating a biological material with use of a clearing reagent further containing aminoalcohol and a nonionic surfactant (Non-Patent Literature 6).

M. Ueda et al. discuss effects of hydrophilic additives on the solubility of nonpolar substances in their publication "Effect of sorbitol and inositol on the critical micelle concentration of nonionic surfactants in water and in aqueous urea", COLLOID & POLYMER SCIENCE, vol. 257, no. 9, September 1979 (1979-09), pages 973-976, XP055508244, ISSN: 0303-402X, DOI: 10.1007/ bf01520723. The cmc of the surfactants was determined using a fluorescent probe as an indicator.

### Citation List

[Patent Literature 1]
   U.S. Patent No. 6472216 (Date of patent: October 29, 2002)
[Patent Literature 2]
   PCT International Publication, No. WO2011/111876 (Publication Date: September 15, 2011)
[Patent Literature 3]
   PCT International Publication, No. WO2013/191274 (Publication Date: December 27, 2013)

### [Non-patent Literature]

[Non-patent Literature 1]
   Ann-Shyn Chiang et al.: Insect NMDA receptors mediate juvenile hormone biosynthesis. PNAS 99 (1), 37-42 (2002).
[Non-patent Literature 2]
   Hans-Ulrich Dodt et al.: Ultramicroscopy: three-dimensional visualization of neuronal networks in the whole mouse brain. Nature Methods 4 (4), 331-336 (2007).
[Non-patent Literature 3]
   Hama et al.: Scale: a chemical approach for fluorescence imaging and reconstruction of transparent mouse brain. Nat Neuroscience 14 (11), 1481-8 (2011).
[Non-patent Literature 4]
   Ke et al.: SeeDB: a simple and morphology-preserving opticalclearing agent for neuronal circuit reconstruction. Nat Neuroscience 16 (8), 1154-61 (2013).
[Non-patent Literature 5]
   Chung et al.: Structural and molecular interrogation of intact biological systems. Nature 497 (7449), 332-7 (2013).
[Non-patent Literature 6]
   Susaki et al.: Whole-brain imaging with single-cell resolution using chemical cocktails and computational analysis. Cell 157 (3), 726-39 (2014).

### Summary

In the past, preserving an original form of a biological material and reducing the amount of time for increasing transparency (light-transmitting property) were incompatible and could not be simultaneously achieved during clearing of the biological material.

For example, a technique (Scale B4) disclosed in WO2012/147965 enables a reduction in the amount of time required for increasing transparency of a biological material. However, if emphasis is placed on rapid permeation of urea or the like which is an active component for clearing treatment, then it results in treatment with urea at a high concentration. This causes expansion (deformation) of a biological material.

While the technique (Scale U2) disclosed in WO2011/111876 effectively suppresses expansion of a biological material, a reduction in the amount of time for increasing transparency is not achieved.

Preserving an original form of a biological material and reducing the amount of time for increasing transparency (light-transmitting property) have thus not been achieved simultaneously.

While CUBIC reduces the amount of time for increasing transparency, CUBIC poses a risk of disrupting the structure of cellular tissue, depending on the effect of a nonionic surfactant contained at a high concentration. In addition, since CUBIC solution containing aminoalcohol has a pH of 11.4, strong alkali may cause denaturation or loss of proteins which constitute membranes. This makes it highly probable that normal fine structures of membranes which constitute cells are not maintained. In fact, the inventors of the present invention observed fine structures with use of an electron microscope, and found that shapes of tissue were seriously disrupted in the cells of a biological material treated by CUBIC. With this knowledge, the inventors of the present invention found that the effect of CUBIC to reduce the amount of time for a clearing treatment is the result of the following: denaturation of the biological material caused destruction of the fine structures of the cells, so that permeation of the clearing reagent became easy.

Therefore, there has not been a clearing reagent which, for preserving an original form of a biological material, does not cause the shapes of cells in the biological material to considerably change at a fine structure level and which enables quick clarification.

The present invention has been made in order to solve the problems, and an object of the present invention is to provide (i) a novel clearing reagent for making a biological material transparent which clearing agent takes advantage of a synergistic effect of, as an active component, a combination of urea or a urea derivative with sorbitol and (ii) use of the clearing agent.

In order to solve the problems, the inventors of the present invention conducted diligent study. First, study was conducted on an active component that increases transparency in a case where the active component permeated into a biological material. It was then found that sorbitol, which by itself was not found substantially effective, brought about a remarkable effective of increasing transparency when used in combination with urea or a urea derivative. Sorbitol causes shrinkage of a biological material. Therefore, the combination of sorbitol with urea, unlike Scale A2 and Scale U2, brought about the effect of preventing expansion of a biological material. It was also found that in a case where the clearing reagent was used for clarification of a biological material in which fluorescent protein was expressed, the clearing reagent causes less quenching of fluorescence of the fluorescent protein in comparison with existing clearing reagents and is therefore suitable for observation of a biological material with use of fluorescent protein.

Then, in view of a method for producing the effect of further reducing the amount of time for increasing transparency, the inventors of the present invention conceived an idea of making a clearing reagent permeate into a biological material which has once been treated to shrink, while the volume of the biological material is restored by stress after removing the shrinkage effect, so that the clearing reagent to efficiently permeates into the biological material without destruction of fine structures of the biological material. This allowed for efficient permeation of a clearing reagent without a chemical treatment that induces (i) denaturation of protein and/or (ii) disruption of fine structures of cells.

The present invention was thus conceived.

Specifically, in order to attain the object, the present invention includes the following configurations. The specific and detailed characteristics of the present invention are defined in independent claim 1. Further embodiments are defined in the dependent claims.

A clearing reagent for making a biological material transparent, including: at least one compound selected from the group consisting of urea and a urea derivative; sorbitol; and a surfactant which is contained at a concentration of 5 (w/v)% or less, the clearing reagent being a solution.

### Advantageous Effects of Invention

The present invention includes a clearing reagent for making a biological material transparent, which clearing reagent uses a combination of urea or a urea derivative with sorbitol. The present invention can advantageously provide (i) a clearing reagent capable of quickly increasing transparency without considerably changing an original form of a biological material and (ii) use of the clearing reagent.

### Description of further embodiments

Following, embodiments, by way of example, are described with reference to figures. In the figures show:

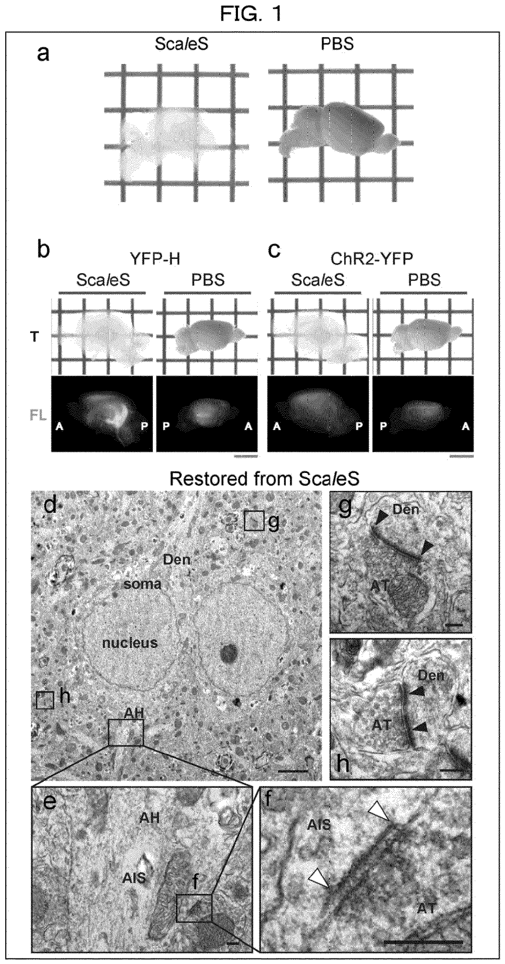

- Fig. 1: illustrates optical clearing and signal / structure preserving capabilities of ScaleS. (a) of Fig. 1 illustrates whole mouse hemispheres (10 weeks old) after treatment with ScaleS (left) and PBS (right), and is based on photographs taken with a patterned background (graph paper ruled into 2.5-mm squares). (b) and (c) of Fig. 1 are images comparing the clarification (T: transmission) and preservation of YFP fluorescence (FL) between ScaleS- (left) and PBS- (right) treated hemispheres from a 10-week-old YFP-H mouse (b) and a 10-week-old ChR2-YFP mouse (c). Because protein leakage varies depending on a degree of tissue fixation, protein leakage may be varied with the individual animal. Direct comparison was herein made between ScaleS and PBS with use of the left hemisphere and the right hemisphere of the same mouse brain. A: (anterior), P: (posterior) Scale bars: 2.5 mm. Incubation time for clearing was optimized according to the size of samples. Transmission images were acquired with the same patterned background as (a). (d) through (h) of Fig. 1 show the results of TEM observation of a sample whose pre-ScaleS treatment state was restored after the treatment. The whole hemisphere of a YFP-H mouse (10 weeks old) was fixed with 4% PFA, and cleared completely by ScaleS. The clearing sample was incubated in the mounting solution (ScaleS4) at 37°C for 14 hours on the assumption that it was subjected to an overnight imaging experiment. Then, the pre-ScaleS treatment state of the clearing sample was returned (restored) by washing the clearing sample with PBS. Then, a 1-mm cube was taken out from the restored sample and used for the preparation of ultrathin sections. (d) of Fig. 1 is a view illustrating a micrograph containing two somata of pyramidal neurons in cortical layer II/III. (e) of Fig. 1 is a view illustrating a magnified micrograph corresponding to the box shown in (d). (f) of Fig. 1 is a magnified micrograph corresponding to the box shown in (e). A symmetric synapse is highlighted by open arrowheads. (g) and (h) of Fig. 1 are views illustrating excitatory, asymmetric synapses in the two regions (boxes in (d)) in the neuropil. Solid arrowheads indicate thickened postsynaptic densities. Den indicates dendrite, AH indicates axon hillock, AIS indicates axon initial segment, and AT indicates axon terminal. Scale bar for (e) is 3 µm, and scale bars for (f), (f'), (g), and (h) are 200 nm.

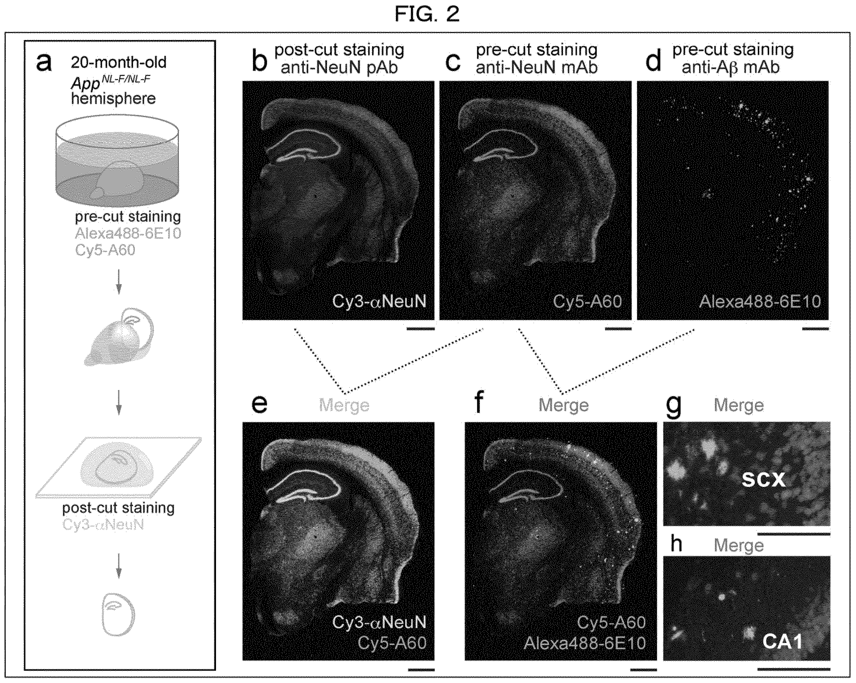

- Fig. 2: illustrates the results of application of AbScale to the left hemisphere of a brain of an aged APP knock-in mouse AppNL-F/NL-F (20 months old) for visualizing Aβ amyloidosis. The maximum size of the hemisphere was 6 mm long, 4 mm wide, and 4 mm high. (a) of Fig. 2 shows schematic diagrams illustrating the triple-color immunohistochemistry. Pre-cut staining of the whole hemisphere was performed with use of anti-Aβ mAb (Alexa488-6E10, shown in cyan color) and anti-NeuN mAb (Cy5-A60, shown in magenta color). Post-cut staining of an excised section was performed with use of anti-NeuN pAb (Cy3-αNeuN, shown in yellow color). For clarity, the hippocampal cell layers are highlighted; the cell layers are displayed in magenta color after pre-cut staining, and then displayed in mixed color of yellow and magenta after post-cut staining. (b) through (d) of Fig. 2 are views illustrating fluorescence images stained in the following colors. (b) of Fig. 2 illustrates post-cut stained with Cy3-βNeuN (displayed in yellow), (c) of Fig. 2 illustrates pre-cut stained with Cy5-A60 (displayed in magenta), and (d) of Fig. 2 illustrates pre-cut stained with Alexa488-6E10 (displayed in cyan). Whereas Cy5-A60 reacted to the C-terminus of NeuN, Cy3-αNeuN reacted to the entire protein. Therefore, no competition occurred between the two antibodies. As illustrated in (c) of Fig. 2, the fluorescence signal intensities for individual neuronal nuclei were nearly constant inside the section regardless of the distance from the brain surface and the median plane. This indicates efficient penetration of Cy5-A60 inside the hemisphere. (e) of Fig. 2 is a merged image of (b) and (c) of Fig. 2. (f) of Fig. 2 is a merged image of (c) and (d) of Fig. 2. (g) and (h) of Fig. 2 illustrates high-magnification images of (f) of Fig. 2. SCX: Somatosensory cortex. All scale bars represent 1 mm.

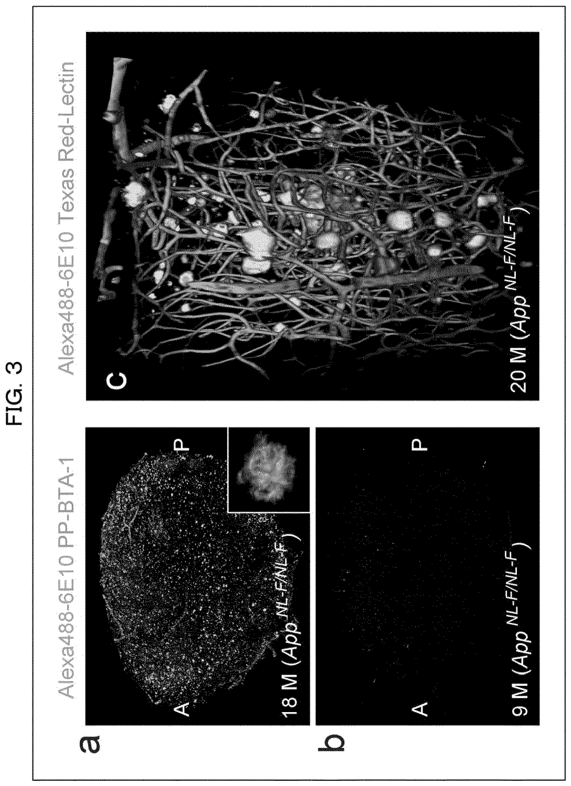

- Fig. 3: illustrates the results of application of combined methods of ChemScale with Ab-Scale to 3D visualization of Aβ plaques. (a) and (b) of Fig. 3 are views illustrating 3D visualization of Aβ plaques in the entire hemispheres of AppNL-F/NL-F mice at 18 months old (a) and 9 months old (b). The hemispheres were stained with PP-BTA-1 (red) and Alexa488-6E10 (green). An Olympus FV1000 system equipped with a 10x objective lens was used to observe the cleared hemispheres from their lateral surfaces and volume rendering images aimed toward the mid-plane. Although it appears that the plaques were distributed throughout the hemisphere in (a) of Fig. 3, observation from different angles (data not shown) revealed that they were mostly located in the cerebral cortex. A indicates anterior, and P indicates posterior. The inset in (a) is a view illustrating a high-magnification volume rendering image of a representative senile plaque. (c) of Fig. 3 is a view illustrating 3D visualization of Aβ plaques (Alexa488-6E10, green) and blood vessels (Texas Red lectin, red) in a part of the cerebral cortex of a 20-month-old AppNL-F/NL-F mouse. A ZEISS Lightsheet Z.1 equipped with a 20x objective lens was used for the observation. Backward and forward perspective images were created from a plurality of images captured at different depths and from different angles.

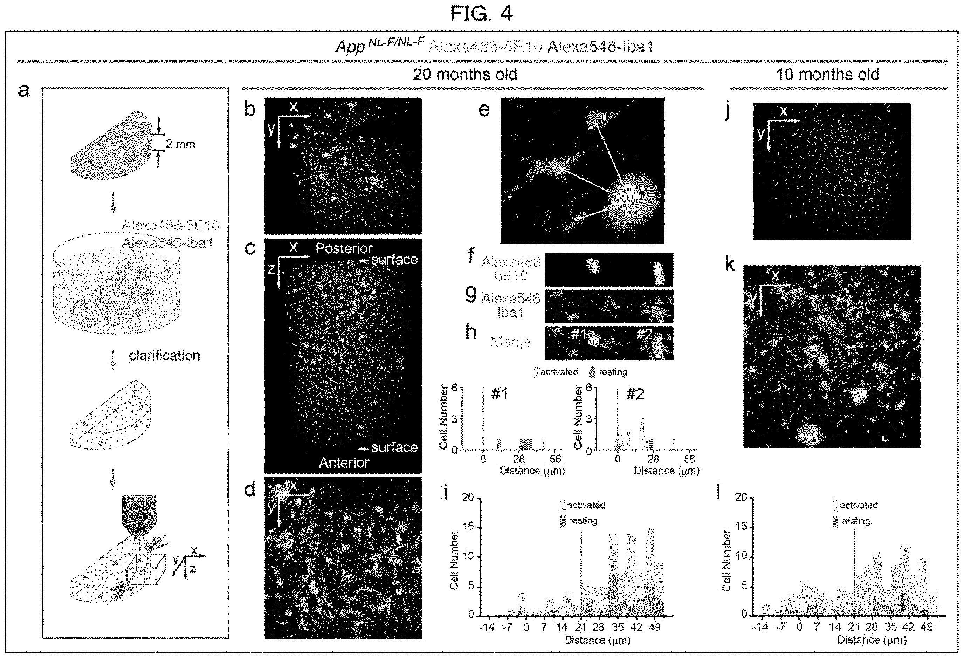

- Fig. 4: is a view made by application of dual-color AbScale to 3D visualization of interactions between Aβ plaques and microglia. All data were obtained by 3D-IHC with use of brain slices of aged AppNL-F/NL-F mice immunostained with Alexa488-6E10 and Alexa546-lba1 (rabbit anti-lbal pAb plus anti-rabbit IgG Ab labeled with Alexa546) 6E10-positive Aβ plaques and Iba1-positive microglia are colored in green and red, respectively. (a) of Fig. 4 is a set of schematic diagrams illustrating how Aβ plaques and microglia inside a 2-mm-thick brain slice are immunostained and how the Aβ plaques and microglia are then observed and imaged with use of the SPIM system. Aβ plaques and microglia are indicated by green spots and red dots, respectively. Cyan and orange arrows indicate a direction of illumination and a direction of detection, respectively, of the SPIM system. The actual observed region is boxed. The xyz coordinates are defined with respect to the objective. (b) through (i) of Fig. 4 are data in which a 20-month-old AppNL-F/NL-F mouse was used. (b) and (c) of Fig. 4 are two volume rendering images generated from the observed region ((b), xy plane; (c), xz plane; see the xyz coordinates in (a)). (d) of Fig. 4 is a high-magnification volume rendering image of 37 Aβ plaques and microglia detected in the observed region. (e) of Fig. 4 is a view illustrating plaque-centered distance measured in 3D space. Automatically calculated distances (yellow arrows) from the plaque edge to individual microglial centers are shown. (f) through (h) of Fig. 4 illustrate two Aβ plaques that differentially interact with microglia. (h) of Fig. 4 is a set histograms illustrating the plaque edge-microglial center distances for the two plaques. The numbers of activated (pink) and resting (violet) microglial cells are plotted. (i) of Fig. 4 is a set of histograms showing distances from the plaque edge to neighboring microglial center for all the 37 plaques. (j) through (I) of Fig. 4 are data in which a 10-month-old AppNL-F/NL-F mouse was used. (j) of Fig. 4 is a view illustrating a volume rendering image generated from the observed region (xy plane). (k) of Fig. 4 is a high-magnification volume rendering image of 27 Aβ plaques and microglia detected in the observed region. (I) of Fig. 4 is a set of histograms showing distances from the plaque edge to neighboring microglial center for all the 27 plaques.

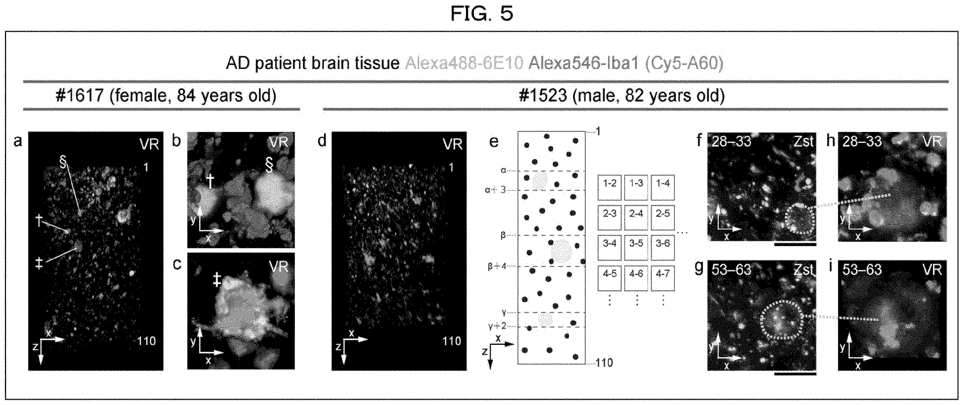

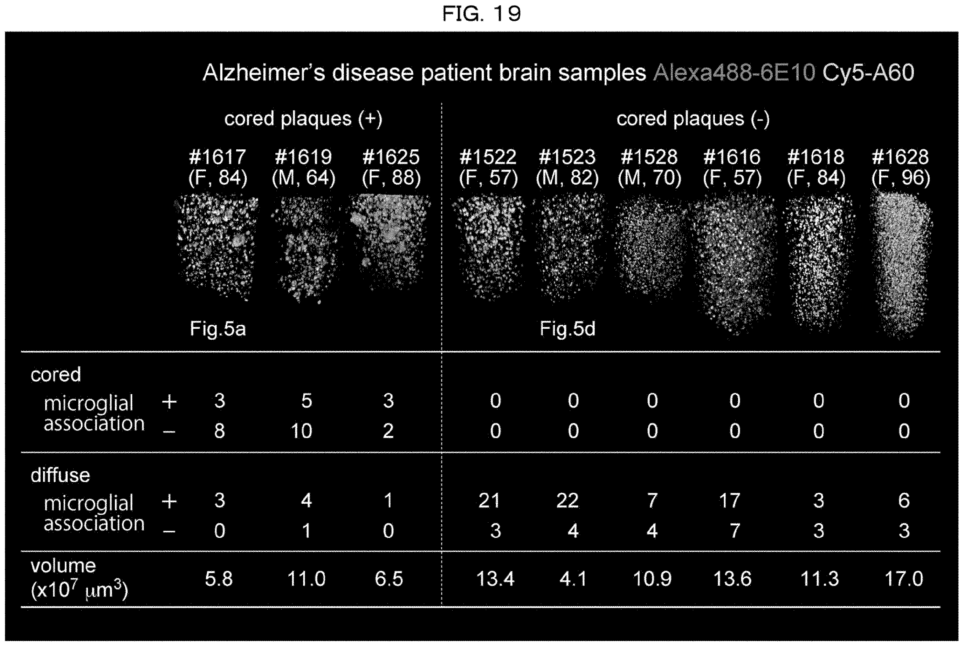

- Fig. 5: is a view made by application of multi-color AbScale to 3D visualization of interactions between Aβ plaques and microglia in the postmortem brain samples of (two) AD patients. Data were obtained by 3D-IHC with Alexa488-6E10, Alexa546-lba1, and Cy5-C60. (a) through (c) of Fig. 5 show data obtained from samples of an 84-year-old woman (identification number #1617). (a) of Fig. 5 illustrates a volume rendering image (xz plane) obtained from the observed region. Eleven cored plaques were found in the region, among which three were associated with and eight were isolated from microglia (see Fig. 19). (b) of Fig. 5 illustrates a high-magnification volume rendering image (xy plane) highlighting two of the isolated plaques (labeled with § and † in (a)). (c) of Fig. 5 illustrates a high-magnification volume rendering (xy plane) highlighting one of the microglia-associated plaques (labeled with ‡ in (a)). (d) through (i) of Fig. 5 show data obtained from samples of an 82-year-old man (identification number #1523). (d) of Fig. 5 is a view illustrating a volume rendering image generated from the observed region (xz plane). (e) of Fig. 5 is a set of schematic diagrams illustrating how the observed region 3D reconstruction composed of 110 xy images is systematically re-sliced to create z-stacked images (2D projection images) of different sites and thicknesses. Relatively large but obscure objects (diffuse plaques) can be seen against abundant bright objects (intracellular 6E10 signals) by z-stacking several xy images. (f) of Fig. 5 illustrates a z-stacked image composed of xy images of image number 28-33 showing the presence of a diffuse plaque that was spatially associated with microglial clustering. (g) of Fig. 5 illustrates a z-stacked image composed of xy images of image number 53-63 showing the presence of a diffuse plaque that was tightly associated with microglia. The systematic re-slicing uncovered a total of 26 diffuse plaques in the observed region shown in (d). See Fig. 19. (h) and (i) of Fig. 5 are volume rendering images that highlight the diffuse plaques shown in (f) and (g) of Fig. 5, respectively. Scale bars for (f) and (g) are 100 µm. VR: volume rendering. Zst: z-stacked.

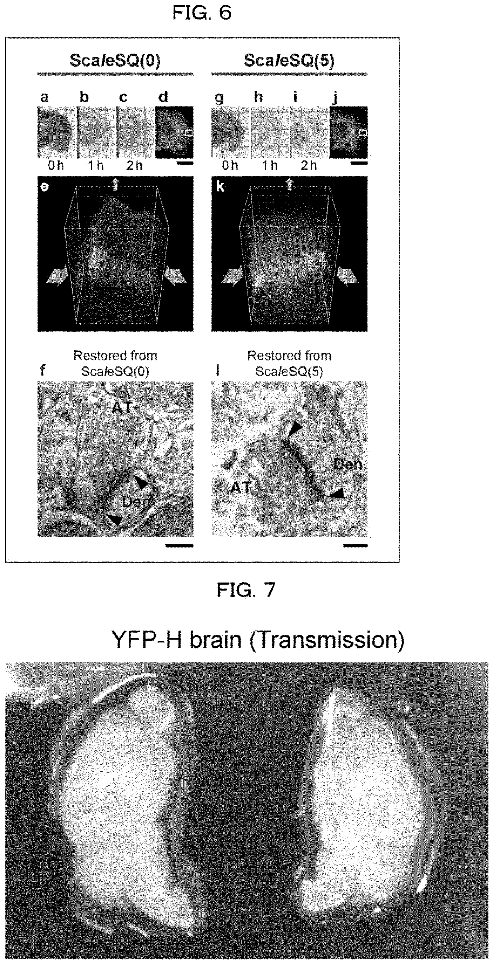

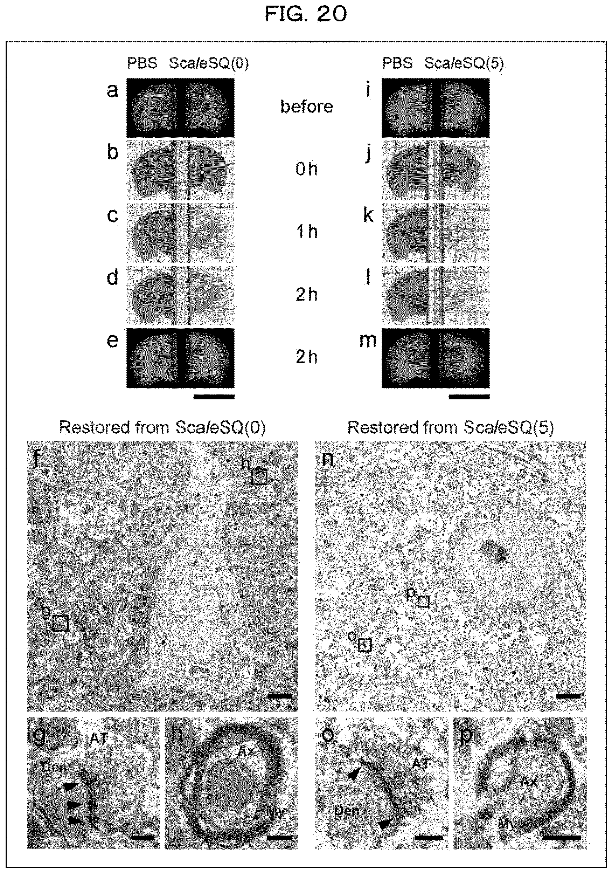

- Fig. 6: illustrates ScaleSQ methods, that is, quick versions of ScaleS applicable to brain slices. Any mounting for microscopic observation was performed by a treatment with use of ScaleS4(0) at room temperature for 2 hours or less. (a) through (f) of Fig. 6 are views illustrating the results of the treatment with use of ScaleSQ(0). (a) through (c) of Fig. 6 illustrate transmission images of a 1-mm-thick brain slice prepared from a 8-week-old YFP-H mouse after 0-hour incubation (a), 1-hour incubation (b), and 2-hour incubation (c) in ScaleSQ(0) at 37°C. (d) of Fig. 6 is a view illustrating fluorescence image of the ScaleSQ(0)-treated brain slice. (e) of Fig. 6 illustrates 3D rendering of YFP-expressing neurons in the cortical region (indicated by a white box in (d)). 723 SPIM images were acquired to make the 3D reconstruction (region delineated by white lines). Blue arrows indicate a direction of illumination, and a green arrow indicates a direction in which fluorescence is detected. (f) of Fig. 6 shows the results of observation of excitatory synapse by EM in a mouse brain sample whose state before ScaleSQ(0) treatment was restored after the treatment. (g) through (I) of Fig. 6 show the results of the treatment with use of ScaleSQ(5). (g) through (i) of Fig. 6 illustrate transmission images of a 1-mm-thick brain slice prepared from a 8-week-old YFP-H mouse after 0-hour incubation (g), 1-hour incubation (h), and 2-hour incubation (i) in ScaleSQ(5) at 37°C. (j) of Fig. 6 is a view illustrating fluorescence image of the ScaleSQ(5)-treated brain slice. (k) of Fig. 6 illustrates 3D rendering of YFP-expressing neurons in the cortical region (indicated by a white box in (j)). 769 SPIM images were acquired to make the 3D reconstruction (region delineated by white lines). (I) of Fig. 6 shows the excitatory synapse observed by EM in a mouse brain sample whose state before ScaleSQ(5) treatment was restored by replacement by PBS after the treatment. Blue and green arrows in (e) and (k) of Fig. 6 indicate directions of illumination and fluorescence detection, respectively. AT indicates axon terminal, and Den indicates dendrite ((f) and (I)). Solid arrowheads indicate thickened postsynaptic densities. Scale bars in (d) and (j) represent 2.5 mm, and scale bars in (f) and (I) represent 200 nm. More detailed information is shown in Fig. 20.
- Fig. 7: is a view in which a comparison of mouse cerebral hemisphere clearing is made with use of (i) a clearing reagent containing sorbitol and (ii) a comparative reagent containing erythritol.

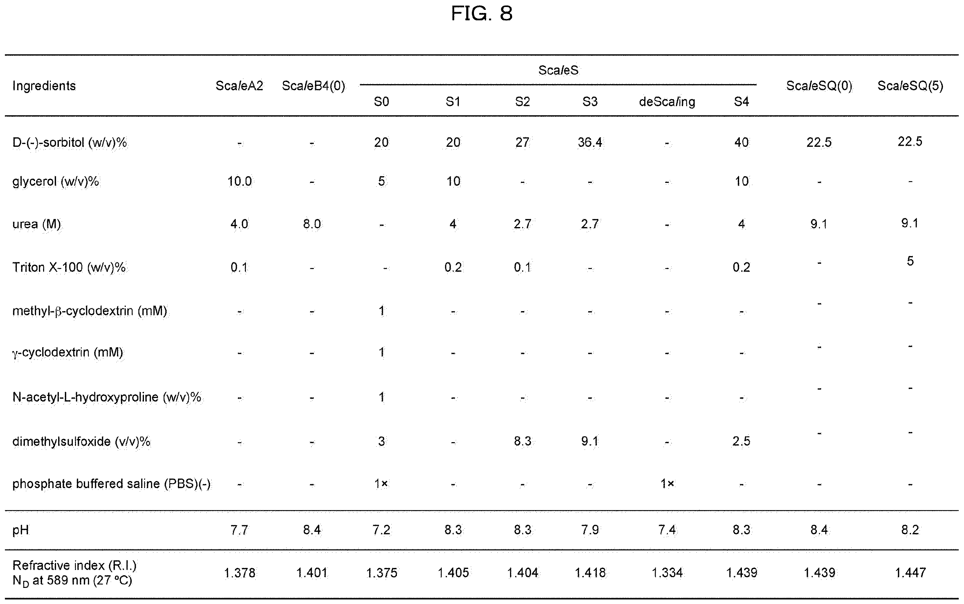

- Fig. 8: is a view illustrating the compositions of the reagents used in example.

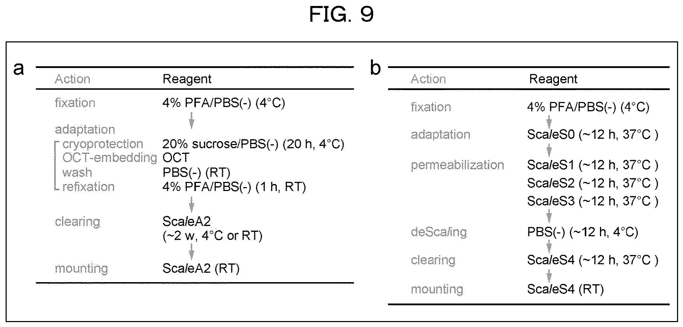

- Fig. 9a: is a view illustrating the protocol of the ScaleA2 treatment. Regarding the protocol, reference can be made to the U.S. patent application publication No. 2013-0045503.
- Fig. 9b: is a view illustrating the protocol of the ScaleS treatment. The composition of a solution for use in the ScaleS treatment is shown in Fig. 8. First, such an extremely easy treat as incubate with use of a ScaleS0 solution is performed to largely increase permeability of a biological material (particularly fixed biological material). Then, the biological material whose permeability has been increased is subjected to incubation sequentially in a ScaleS1 solution, a ScaleS2 solution, and a ScaleS3 solution. Lastly, the biological material is washed with use of PBS (deScaling), an original state of the biological material is restored. Then, the biological material is subjected to incubation in a ScaleS4 solution before observation. The ScaleS4 solution can be used alternatively as a mounting solution. Incubation in each stage can be performed at a low temperature of approximately 4°C. Note, however, that by performing the incubation at a high temperature (e.g. 37°C) as illustrated, it is possible to obtain the effect of further accelerating the clearing treatment. Incubation is performed in an orbital shaker capable of controlling temperatures.

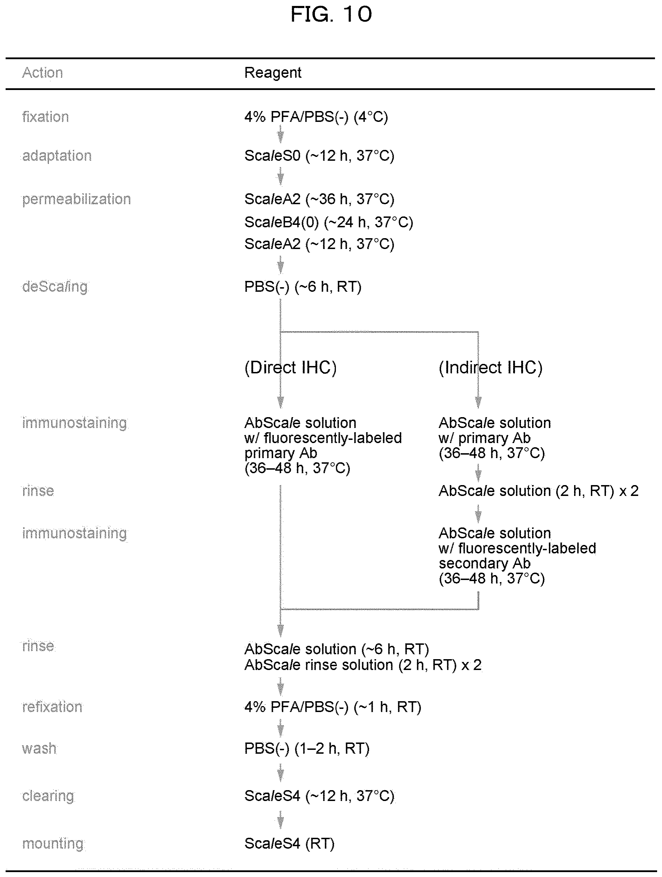

- Fig. 10: is a view illustrating the protocol of AbScale. Note that ScaleB4(0) illustrated in Fig. 10 is an aqueous urea solution at a concentration of 8 M.

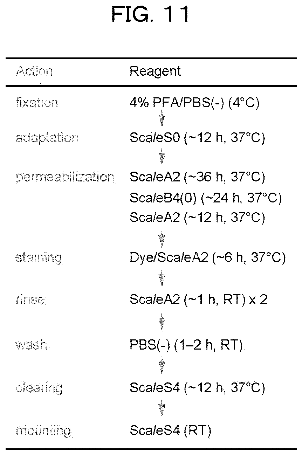

- Fig. 11: is a view illustrating the protocol of ChemScale. Note that the composition of ScaleB4(0) illustrated in Fig. 11 is identical to that of Fig. 10.

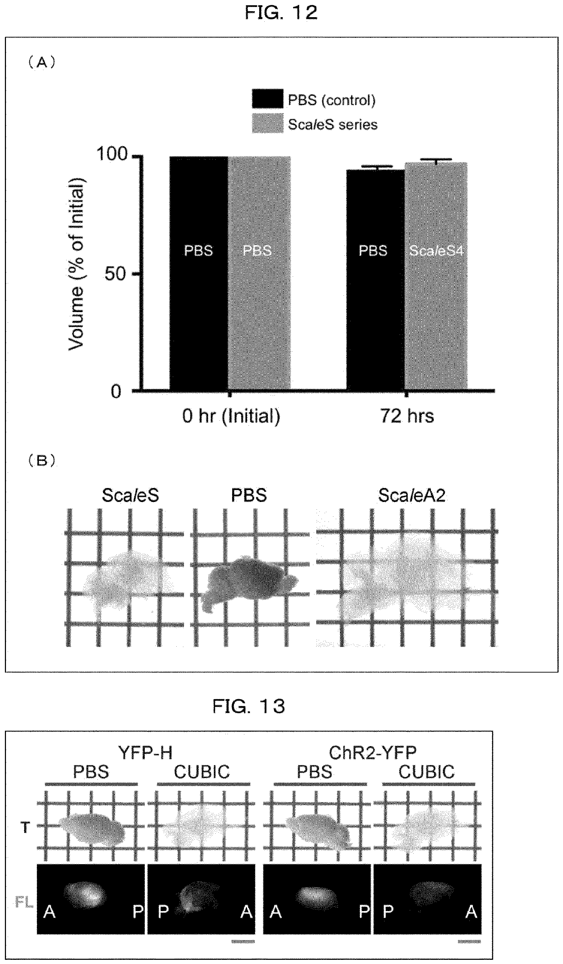

- Fig. 12: is a set of views illustrating a change of volume of a biological material through a ScaleS treatment, (A) of Fig. 12 being a graph comparing between volumes before the treatment (0 hours) and after the treatment (72 hours) (the control is PBS treatment) and (B) of Fig. 12 illustrating appearances of the biological material after the ScaleS treatment, the ScaleA2 treatment, and the PBS treatment (control).
- Fig. 13: is a view illustrating adverse effect of a CUBIC-based treatment on YFP fluorescence in experiments in which two mouse-derived samples were used. The CUBIC-based treatment causes reduction of YFP fluorescence more than the PBS treatment (control).

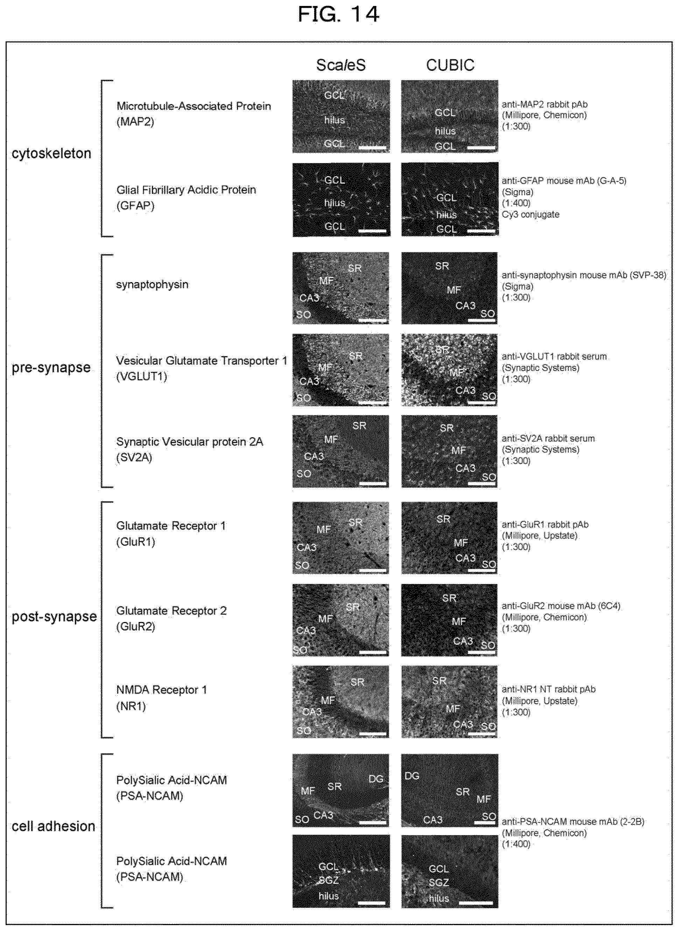

- Fig. 14: illustrates immunohistochemistry of sections which were restored to the original states after the ScaleS treatment (on the left) and the CUBIC treatment (on the right). The brain samples obtained from C57BL6/J wild-type mouse (10 weeks old) were split into two hemispheres. The right hemisphere and the left hemisphere were cleared by the ScaleS treatment and the CUBIC treatment, respectively, and then washed with PBS, so that the original states were restored. After cryoprotection in 20% sucrose/PBS, the restored brain samples were embedded in an OCT compound. Cryostat was used to cut out coronal sections having a thickness of 50 µm. The sections thus obtained were subjected to a permeabilizing treatment/a blocking treatment in a 0.1% Triton X-100 (wt/vol)/ 1% Blocking reagent (Roche)/PBS for 1 hour. Then, the resulting sections were treated by free floating immunohistochemistry. The secondary antibodies used were: a goat antibody to rabbit IgG conjugated with Alexa Fluor546 (Molecular Probes); a goat antibody to mouse IgG conjugated with Alexa Fluor546 (Molecular Probes); and a goat antibody to rabbit IgG conjugated with Alexa Fluor488 (Molecular Probes). DG indicates dentate gyrus, GCL indicates granule cell layer, MF indicates mossy fiber, SGZ indicates subgranular zone, SO indicates stratum oriens, and SR indicates stratum radiatum. Scale bars are 100 µm.

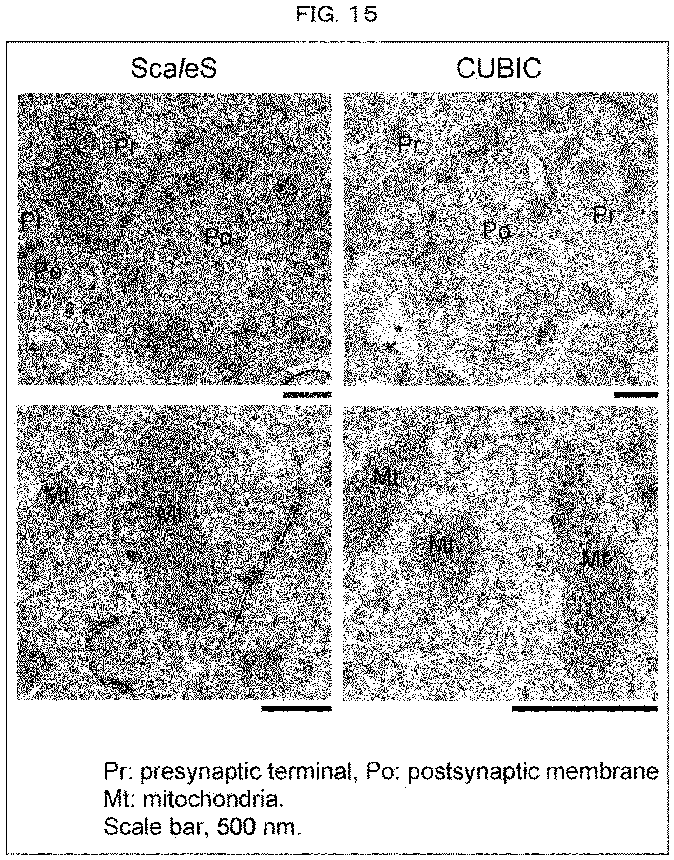

- Fig. 15: shows the results of TEM observation of brain samples which were restored to the original states after ScaleS treatment (on the left) and the CUBIC treatment (on the right). The whole brain of a C57BL6/J mouse (9 weeks old) was fixed with 4% PFA. A slice (1-mm-thick) containing the hippocampus was prepared from the brain thus fixed, and was split into halves. The left half was cleared by a ScaleS treatment according to the method illustrated in b of Fig. 9. The right half was cleared by CUBIC, and, in view of the thickness of the slice, the amount of time for incubation with a CUBIC Reagent 1 was reduced to a half. The two cleared samples were washed with PBS (-) at 4°C for 12 hours, so that the original states were restored. Ultrathin sections were prepared from the two restored slices, and then subjected to TEM observation with use of 1200EX-II (JEOL).

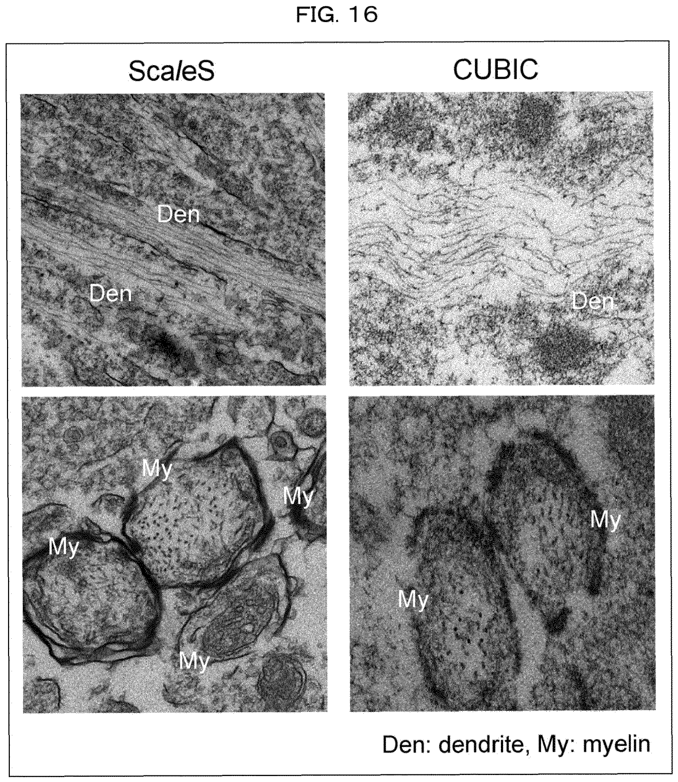

- Fig. 16: is another view showing the results of TEM observation of brain samples which were restored to the original states after ScaleS treatment (on the left) and the CUBIC treatment (on the right) as with the results shown in Fig. 15.

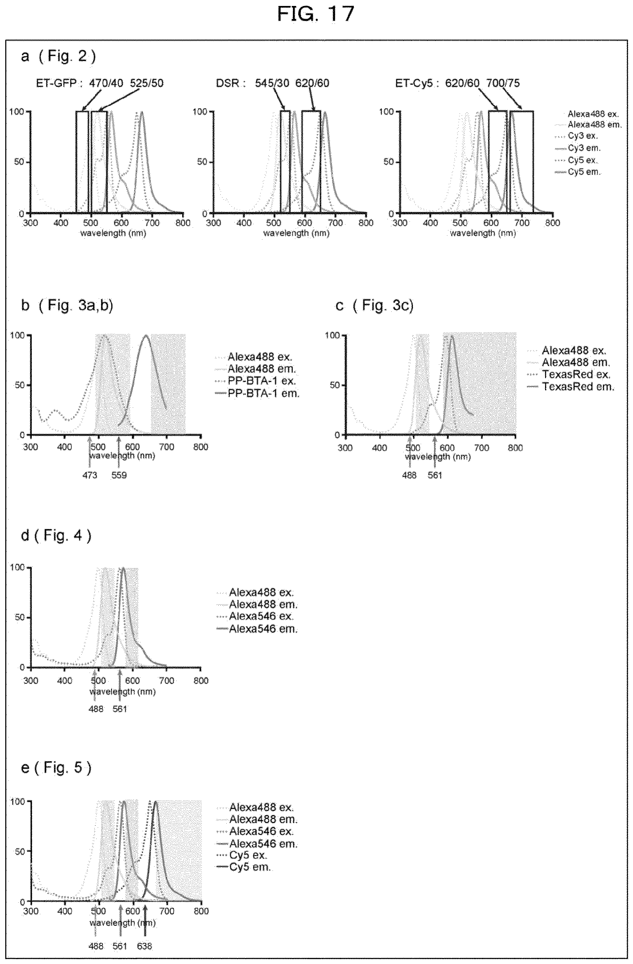

- Fig. 17: is a view illustrating optical elements used for multi-color imaging experiments. Excitation spectrums (dotted lines) and emission spectrums (solid lines) of Alexa488, Alexa546, Cy3, Cy5, and PP-BTA-1 are normalized, and indicated in the same colors as corresponding ones of Fig. 2, Fig. 3, Fig. 4, and Fig. 5. (a) of Fig. 17 illustrates observation with use of a stereomicroscope (corresponding to Fig. 2). Transmission characteristics of excitation filters (for stereomicroscope) and emission filters are delineated in boxes. (b), (c), and (d) of Fig. 17 illustrate observation with use of a confocal microscope (corresponding to a of Fig. 3 and b of Fig. 3) and observation with use of SPIM (c of Fig. 3, Fig. 4 and Fig. 5). In Fig. 17, laser lines are indicated by arrows. Transmission characteristics of emission filters are indicated by green and red boxes.

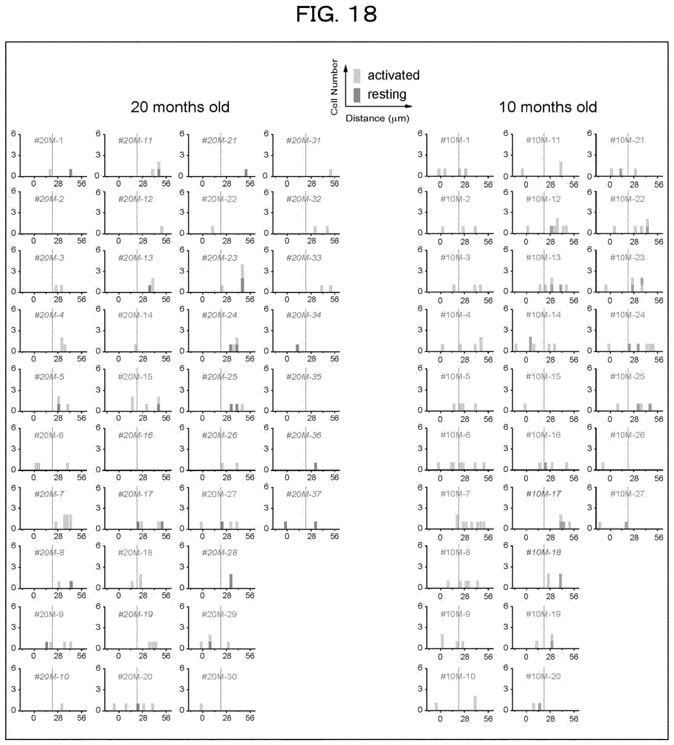

- Fig. 18: is a bar graph showing distributions, for individual plaques, of distances of activated microglia and resting microglia to plaque edge. The left side of Fig. 18 shows 37 plaques in a brain region of a 20-month-old APPNL-F/NL-F mouse. The right side of Fig. 18 shows 27 plaques in a brain region of a 10-month-old APPNL-F/NL-F mouse. The obsolete plaques which are not associated with any activated microglia in their vicinity (closer than a distance of 21 µm) are numbered in light-color writing. The other plaques (presumably acute or subacute) are numbered in red. The vertical dotted lines in the graph are located at 21 µm to allow close neighboring microglia and far neighboring microglia to be distinguished.
- Fig. 19: is a view illustrating 3D reconstruction of two immunosignals (6E10 (green) and A60 (blue)) in postmortem brain samples of an Alzheimer's Disease patient. In each sample, cored plaques and diffuse plaques when there is association with microglia and when there is no association with microglia were counted, and the volumes thereof were measured. In addition, microglial cells of all of the samples were immunostained. The 6E10 signal (green) and Ibal signal (red) for #1617 and #1523 are shown in (a) of Fig. 5 and (b) of Fig. 5, respectively.
- Fig. 20: is a view showing the results of treatments with ScaleSQ(0) ((a) through (h)) and ScaleSQ(5) ((i) through (p)). (a) through (h) of Fig. 20 are fluorescence images ((a) and (e)) and transmission images ((b) through (d)) of 1-mm-thick brain sections of an 8-week-old YFP-H mouse before incubation in a 37°C ScaleSQ(0) (a), 0 hours later (b), 1 hour later (c), and 2 hours later ((d) and (e)). (f) through (h) of Fig. 20 show the results of TEM observation of the brain samples restored from the ScaleSQ(0) treatment. Ultrathin sections were produced and imaged as in Fig. 1. (f) of Fig. 20 is a view showing the results of microscopic observation in which a neuronal soma is captured. (g) of Fig. 20 is a view illustrating an enlarged microscopic image corresponding to the boxed portion in (f), which indicates excitatory asymmetric synapse. (h) of Fig. 20 is a view illustrating an enlarged microscopic image corresponding to the boxed portion in (f), which indicates myelinated axon. (i) through (m) of Fig. 20 are fluorescence images ((i) and (m)) and transmission images ((j) through (I)) of 1-mm-thick brain sections of an 8-week-old YFP-H mouse before incubation in a 37°C ScaleSQ(5) (i), 0 hours later (j), 1 hour later (k), and 2 hours later ((I) and (m)). (n) through (p) of Fig. 20 show the results of TEM observation of the brain samples restored from the ScaleSQ(5) treatment. Ultrathin sections were produced and imaged as in Fig. 1. (n) of Fig. 20 is a view illustrating a microscopic image in which a neuronal soma is captured. (o) of Fig. 20 is a view illustrating an enlarged microscopic image corresponding to the boxed portion in (n), which indicates excitatory asymmetric synapse. (p) of Fig. 20 is a view illustrating an enlarged microscopic image corresponding to the boxed portion in (n), which indicates myelinated axon. Arrowheads indicate thickened postsynaptic densities. Den indicates dendrite, AT indicates axon terminal, and My indicates myelin. Scale bars in (a) through (e) and (i) through (m) are 5 mm. Scale bars in (f) and (n) are 4 µm. Scale bars in (g), (h), (o), and (p) are 200 µm.

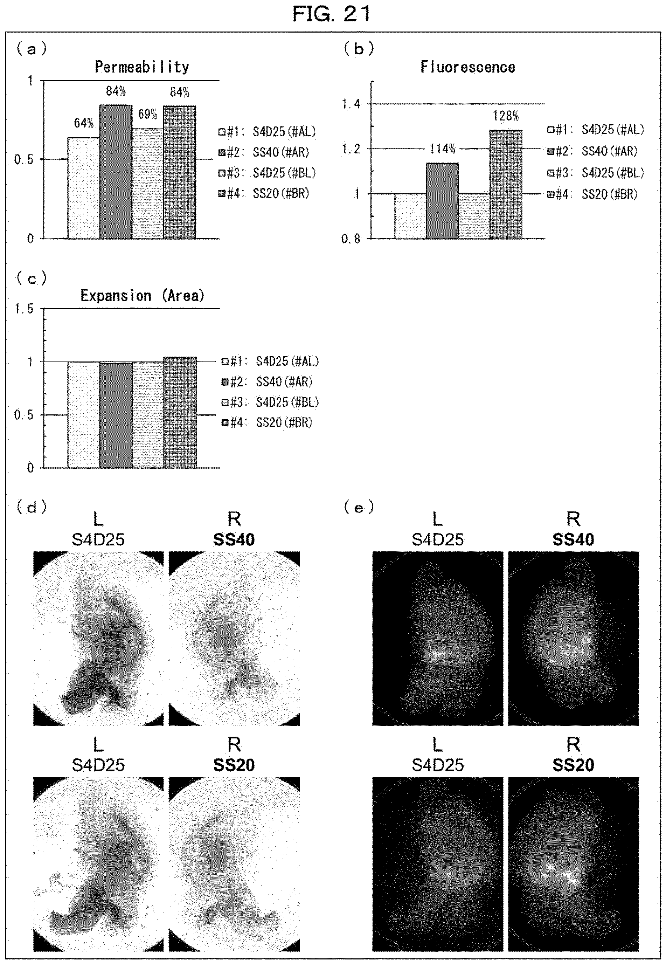

- Fig. 21: is a view showing the results of investigations into the light-transmitting property, the YFP fluorescence signal intensity, and the change of volume of hemispheres of YFP-H mice treated with ScaleSS20 and ScaleSS40. (a) through (c) of Fig. 21 are views each showing, by graph, the light-transmitting property, the YFP fluorescence signal intensity, and the change of volume. (d) through (e) of Fig. 21 are views based on photographs showing the results of clearing with ScaleSS20 and ScaleSS40. (d) of Fig. 21 is a view based on a micrograph. (e) of Fig. 21 shows a fluorescence image of (d). (L) indicates left hemispheres, and (R) indicates right hemispheres.

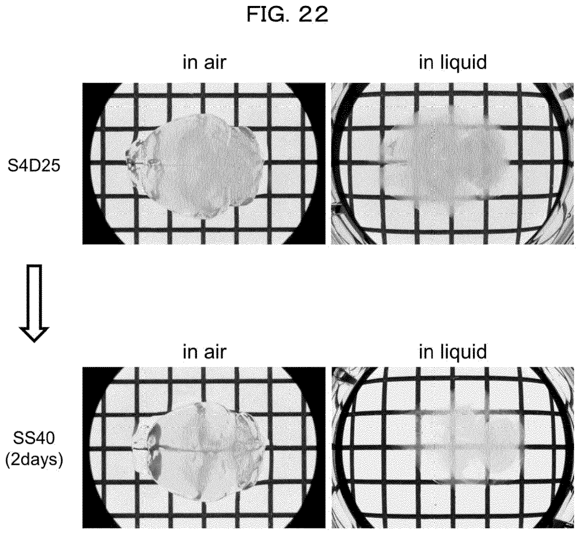

- Fig. 22: is a view based on photographs of a whole brain of a YFP-H mouse which is treated with ScaleSS40 and captured along with patterned backgrounds.

The following description will discuss an embodiment of the present invention in detail.

### [1. Clearing reagent for making biological material transparent]

### (Active component of clearing reagent for making biological material transparent)

A "clearing reagent for making a biological material transparent" in accordance with an aspect of the present invention is a solution containing "urea" as an essential active component.

A "clearing reagent for making a biological material transparent" in accordance with another aspect of the present invention is a solution containing a "urea derivative" as an essential active component.

The urea derivative is not limited to any particular kind. Specifically, for example, the urea derivative is any of various kinds of ureine or compounds expressed by Formula (1) below. Note that the compounds expressed by Formula (1) include part of ureines. The clearing reagent for making a biological material transparent in accordance with an embodiment of the present invention needs to contain, as an active component, at least one compound selected from the group consisting of urea and urea derivatives. Among these, the clearing agent more preferably contains urea.

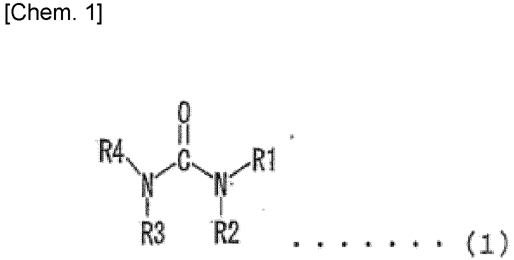

In a urea derivative expressed by Formula (1), each of R1, R2, R3, and R4 is independently a hydrogen atom (note that the one in which all of R1 through R4 are hydrogen atoms is excluded, since it corresponds to urea), a halogen atom, or a hydrocarbon group. Further, in a case where the hydrocarbon group has a plurality of carbon atoms, part of the carbon atoms can be replaced by a hetero atom such as a nitrogen atom, an oxygen atom, or a sulfur atom. Examples of the hydrocarbon group encompass a chain hydrocarbon group and a cyclic hydrocarbon group.

Examples of the chain hydrocarbon group encompass a chain alkyl group, a chain alkenyl group, and a chain alkynyl group. The chain hydrocarbon group can have any number of carbon atoms. For example, the chain hydrocarbon group can be straight-chain or branched one having 6 or less carbon atoms, preferably, an alkyl group having 1 through 3 carbon atoms. The chain hydrocarbon group can have a substituent such as a halogen atom. Examples of the chain alkyl group encompass a methyl group, an ethyl group, a propyl group, an isopropyl group, a butyl group, an iso-butyl group, a sec-butyl group, a tert-butyl group, a hexyl group, and an octyl group.

The cyclic hydrocarbon group can be, for example, a cycloalkyl group or a cycloalkenyl group. The cyclic hydrocarbon group can have a substituent such as a halogen atom. Examples of the cycloalkyl group encompass those having 3 or more and preferably 6 or less carbon atoms, such as a cyclopropyl group, a cyclobutyl group, a cyclopentyl group, and a cyclohexyl group. Examples of the cycloalkenyl group encompass those having 3 or more and preferably not more than 6 carbon atoms, such as a cyclohexenyl group.

Examples of the halogen atom encompass a fluorine atom, a chlorine atom, a bromine atom, and an iodine atom.

The following 1) and 2) are more preferable, specific examples of the urea derivatives expressed by Formula 1:
1) Any three groups selected from R1 through R4 are hydrogen atoms, and the other one group is (i) a halogen atom or (ii) a chain hydrocarbon group having 1 through 6 carbon atoms, more preferably, the other one group is an alkyl group having (i) 1 through 3 carbon atoms or (ii) 1 or 2 carbon atoms.
2) Any two groups selected from R1 through R4 are hydrogen atoms, and each of the other two groups is independently (i) a halogen atom or (ii) a chain hydrocarbon group having 1 through 6 carbon atoms, more preferably, both of the other two groups are alkyl groups each having (i) 1 through 3 carbon atoms or (ii) 1 or 2 carbon atoms. Still more preferably, one of the two groups which are hydrogen atoms is selected from R1 and R2, and the other of the two groups is selected from R3 and R4.

An amount, by which the at least one compound selected from the group consisting of urea and a urea derivative is contained, is not particularly limited, provided that a biological material can be cleared. The compound is contained at a concentration in a range of 3 M or more and 5 M or less, or in a range of 5 M or more and 9.5 M or less The upper limit of the "urea and a urea derivative" content is determined by the solubility of the urea and the urea derivative in a solvent used. For example, a "clearing reagent for making a biological material transparent" with a relatively small amount of urea and a urea derivative can perform a required treatment by performing the treatment for a long time, while a clearing reagent with a relatively large amount of urea and a urea derivative can perform a required treatment by performing the treatment for a short time, although this depends on the type of the biological material to be subjected to the treatment.

### (Sorbitol)

A "clearing reagent for making a biological material transparent" in accordance with an embodiment of the present invention contains, as an essential active component, "sorbitol" in addition to at least one compound selected from the group consisting of urea and a urea derivative. The "clearing reagent for making a biological material transparent" contains sorbitol at a concentration in a range of 30 (w/v)% or more to 50 (w/v)% or less, or in a range of 20 (w/v)% or more to 40 (w/v)% or less. In a case where the sorbitol concentration falls within these ranges in the "clearing reagent for making a biological material transparent" in accordance with an embodiment of the present invention, the at least one compound selected from the group consisting of urea and a urea derivative is contained at a concentration in a range of 3 M or more and 5 M or less, or in a range of 5 M or more and 9.5 M or less, respectively. In particular, in a case where a sorbitol concentration and a urea concentration or the like fall within the ranges described above, a reduction in the amount of time for the clearing treatment and prevention of deformation of the biological material are simultaneously achieved.

### (Advantages of combination of urea or urea derivative and sorbitol used as active components)

An example of an advantage of a combination of urea or a urea derivative and sorbitol to be used is that it is possible to quickly increase transparency of a biological material without largely changing an original form of the biological material, in comparison with a case where only urea or a urea derivative is used as an active component for a clearing treatment. In a case where sorbitol was used apart from urea or a urea derivative, no substantial effect was found. However, in a case where sorbitol was used in combination with urea or a urea derivative, the effect of not largely changing the original form of a biological material and the effect of being able to quickly perform clearing are simultaneously achieved. As a result, even if a biological material is, for example, extremely fragile tissue (e.g. embryo), a clearing treatment can be performed quickly while damage and deformation of the biological material are suppressed. Even in a case of clearing a biological sample derived from an aged organism which is conventionally considered difficult to clear, a clearing treatment can be quickly performed while damage and deformation of the biological sample are suppressed.

Furthermore, urea, a urea derivative, and sorbitol have the following features in common. Urea, a urea derivative, and sorbitol are extremely low toxicity. In particular, urea and sorbitol are ingredients derived from a living organism. Therefore, the "clearing reagent for making a biological material transparent" in accordance with an embodiment of the present invention has a low possibility of damaging fluorescent proteins and quenching of fluorescence therefrom, and therefore the clearing reagent used in the present invention is also applicable to an observation of a biological material with use of a fluorescent protein. 3) Urea, a urea derivative, and sorbitol are extremely inexpensive and easily available, and are easy to handle; therefore, use of the clearing reagent used in the present invention allows a clearing treatment to be performed at an extremely low cost and by a simple procedure.

In addition to the above advantages, in comparison with conventional clearing reagents for making a biological material transparent, the clearing reagent used in an embodiment of the present invention can greatly improve transparency of non-transparent biological materials having high light scattering properties, thereby enabling an observation of various fluorescent proteins and fluorescent substances existing in ultra-deep tissues. Particularly, for brain tissues, use of the clearing reagent in accordance with an embodiment of the present invention makes it possible to clear a white matter layer, which has been a barrier against an observation of a deep part, thereby enabling an observation of a region (e.g. corpus callosum) located deeper than the white matter layer. A clearing treatment using the clearing reagent in accordance with an embodiment of the present invention is reversible. Specifically, merely by immersing in an equilibrium salt solution a biological material having been subjected to the clearing treatment, it is possible to bring the biological material back to a state that the biological material had before the clearing treatment. Further, before and after the clearing treatment, antigenicity of a protein and/or the like is substantially unchanged and preserved. This allows an assay by means of an ordinary tissue staining or immunostaining.

### (Glycerol)

A clearing reagent for making a biological material transparent" in accordance with an embodiment of the present invention contains "glycerol" as necessary. The glycerol content is not particularly limited. Glycerol is to be contained at a concentration preferably in a range of 2.5 (w/v)% or more to 35.0 (w/v)% or less, more preferably in a range of 3.0 (w/v)% or more to 30.0 (w/v)% or less, and particularly preferably in a range of 5.0 (w/v)% or more to 25.0 (w/v)% or less. Note that the unit "(w/v)%" represents a percentage of a weight (w (gram)) of glycerol used, with respect to a volume (v (milliliter)) of a "clearing reagent for making a biological material transparent".

Glycerol is relatively unlikely to be rejected by an immune response. Further, glycerol is relatively unlikely to be accumulated in a liver, a kidney, and the like because the glycerol is unlikely to be trapped by a reticuloendothelial system. Accordingly, the "clearing reagent for making a biological material transparent" is easily applied to a living organism as a biological material. Further, the glycerol has an advantage of being relatively inexpensive.

### (Surfactant)

A clearing reagent for making a biological material transparent" in accordance with an embodiment of the present invention contains a surfactant as necessary. The surfactant is preferably a nonionic surfactant, since the nonionic surfactant gently facilitates intrusion of the present clearing reagent into a biological tissue. Examples of the nonionic surfactant encompass: fatty acid surfactants such as polyoxyethylene sorbitan monolaurate, polyoxyethylene sorbitan monopalmitate, polyoxyethylene sorbitan monostearate, and polyoxyethylene sorbitan monooleate; higher alcohol surfactants such as polyvinyl alcohol; and alkylphenol surfactants such as polyoxyethylene octylphenyl ether. Specifically, for example, the surfactant can be at least one kind selected from the group consisting of: Triton X (Registered Trademark) series such as Triton X-100 and Triton X-140; Tween (Registered Trademark) series such as Tween-20, Tween-40, Tween-60, and Tween-80; and NP-40 (product name). As the surfactant, a mixture of two or more kinds can be used as necessary.

These surfactants can enhance permeability of urea with respect to a biological material, thereby improving efficiency of the clearing treatment. In particular, in a clearing treatment on a biological material (e.g. a white matter layer of brain tissues, a spinal cord, a fiber bundle of peripheral nerves) on which a clearing treatment is relatively difficult, the "clearing reagent for making a biological material transparent" preferably contains a surfactant.

Note that, as with the case of urea, above surfactants can enhance permeability of the urea derivative with respect to a biological material.

In a case where a surfactant is to be used, it is necessary to suppress a surfactant concentration to 5.0 (w/v)% or less so as to prevent denaturation of fine structures of cells in a biomaterial. The surfactant concentration is not particularly limited, provided that the surfactant concentration is 5.0 (w/v)% or less. The surfactant is to be contained at a concentration preferably in a range of 0.025 (w/v)% or more to 2.5 (w/v)% or less, more preferably in a range of 0.05 (w/v)% or more to 0.5 (w/v)% or less, and particularly preferably in a range of 0.05 (w/v)% or more to 0.2 (w/v)% or less. Note that the unit "(w/v)%" represents a percentage of a weight (w (gram)) of a surfactant used, with respect to a volume (v (milliliter)) of a "clearing reagent for making a biological material transparent".

### (Water-soluble macromolecular compound)

A "clearing reagent for making a biological material transparent" in accordance with an embodiment of the present invention can further contain a water-soluble macromolecular compound as necessary. Note that the macromolecular compound refers to the one which has a molecular weight of approximately 50,000 to 60,000 or more, for example, and which substantially does not intrude into cells. Further, the macromolecular compound is preferably the one which does not cause denaturation or the like of a biological material. Specific examples of the water-soluble macromolecular compound encompass a crosslinked sucrose macromolecular substance, polyethylene glycol, polyvinyl pyrrolidone, and Percoll (product name; a macromolecular substance obtained by covering colloidal silica with a polyvinyl pyrrolidone film). Specific examples of the crosslinked sucrose macromolecular substance encompass a macromolecular substance which is obtained by crosslinking (copolymerizing) sucrose with epichlorohydrin and which has a weight-average molecular weight of approximately 70,000, such as Ficoll PM70 (product name).

Unlike urea and a urea derivative, these water-soluble macromolecular compounds do not intrude into cells. Furthermore, these water-soluble macromolecular compounds are soluble in water. Therefore, these water-soluble macromolecular compounds are considered to contribute to controlling of an osmotic pressure difference between the inside and outside of a cell. As such, each of these water-soluble macromolecular compounds helps a biological material to be subjected to a clearing treatment maintain its original shape, and particularly contributes to prevention of expansion of the biological material. In a case where the "clearing reagent for making a biological material transparent" in accordance with an embodiment of the present invention has a relatively high osmotic pressure, the clearing reagent preferably contains any of these water-soluble macromolecular compounds. However, the present invention is not limited to this.

In a case where a "water-soluble macromolecular compound" is used, an amount by which the water-soluble macromolecular compound is contained not particularly limited. The water-soluble macromolecular compound concentration is preferably in a range of 2.5 (w/v)% or more to 40.0 (w/v)% or less. Further, in view of a balance between an expansion prevention effect for a biological material and a refractive index of the biological material after the clearing treatment, the above concentration is more preferably in a range of 5.0 (w/v)% or more to 25.0 (w/v)% or less, still more preferably in a range of 10.0 (w/v)% or more to 20.0 (w/v)% or less, and particularly preferably in a range of 10.0 (w/v)% or more to 15.0 (w/v)% or less. Note that the unit "(w/v)%" represents a percentage of a weight (w (gram)) of a "water-soluble macromolecular compound" used, with respect to a volume (v (milliliter)) of a "clearing reagent for making a biological material transparent".

### (Other component 1)

A "clearing reagent for making a biological material transparent" in accordance with an embodiment of the present invention can contain a "drying inhibition component", which is at least one compound selected from carboxy vinyl polymer, hydroxypropyl methyl cellulose, propylene glycol, and macrogol, as necessary. The drying inhibition component prevents drying of a biological material subjected to a clearing treatment. In particular, in a case where there is a relatively long time between clearing treatment of the biological material and an optical microscopic observation of the biological material, or in a case where an optical microscopic observation of the biological material is time-consuming, the clearing reagent in accordance with an embodiment of the present invention preferably contains any of the above drying inhibition components. Note that the glycerol also produces a drying inhibition effect.

In a case where the "drying inhibition component" is used, an amount by which the drying inhibition component is contained is not particularly limited. The drying inhibition component is to be contained at a concentration preferably in a range of more than 0 (w/v)% to 10.0 (w/v)% or less, more preferably in a range of 1.0 (w/v)% or more to 7.0 (w/v)% or less, and particularly preferably in a range of 2.5 (w/v)% or more to 5.0 (w/v)% or less. Note that the unit "(w/v)%" represents a percentage of a weight (w (gram)) of a "drying inhibition component" used, with respect to a volume (v (milliliter)) of a "clearing reagent for making a biological material transparent".

### (Other component 2)

As necessary, a "clearing reagent for making a biological material transparent" in accordance with an embodiment of the present invention can further contain, as a "transparency increasing component", a compound included in the group consisting of sugar and sugar alcohol which exclude sorbitol. Examples of the compound included in the group consisting of sugar and sugar alcohol which exclude sorbitol encompass, but are not limited to, erythritol, fructose, glycerol, mannitol, sucrose, and xylitol. The transparency increasing component if used alone is inferior in clearing performance as a replacement of sorbitol which is an essential active component in the clearing reagent for making a biological material transparent in accordance with an embodiment of the present invention. However, in a case where the transparency increasing component is used along with a combination of urea or a urea derivative and sorbitol, the transparency increasing component contributes to a further increase in transparency of a biological material to be subjected to a clearing treatment, in comparison with a case where the "transparency increasing component" is not used.

In a case where the "transparency increasing component" is used, an amount by which the transparency increasing component is contained is not particularly limited. The transparency increasing component is to be contained at a concentration preferably in a range of more than 0 (w/v)% to 50.0 (w/v)% or less, more preferably in a range of 10.0 (w/v)% or more to 45.0 (w/v)% or less, and particularly preferably in a range of 20.0 (w/v)% or more to 40.0 (w/v)% or less. Note that the unit "(w/v)%" represents a percentage of a weight (w (gram)) of a "transparency increasing component" used, with respect to a volume (v (milliliter)) of a "clearing reagent for making a biological material transparent".

Further, in addition to the above-described "urea and/or a urea derivative", "surfactant", and "water-soluble macromolecular compound", a "clearing reagent for making a biological material transparent" in accordance with an embodiment of the present invention can contain an additive(s) such as a pH adjusting agent and/or an osmotic pressure controlling agent, as necessary.

As necessary, the "clearing reagent for making a biological material transparent" in accordance with an embodiment of the present invention can further contain a compound which is selected from the group consisting of cyclodextrins and/or collagen binding compound (N-acetyl-L-hydroxyproline and the like). Cyclodextrins is a term by which to collectively refer to compounds each of which has a cyclodextrin skeleton which is formed by cyclically linked plural glucoses. More specifically, examples of the cyclodextrins encompass α-cyclodextrin, a derivative of α-cyclodextrin, β-cydodextrin, a derivative of β-cydodextrin (such as methyl-β-cyclodextrin), γ-cyclodextrin, and a derivative of γ-cyclodextrin. An amount by which cyclodextrin is contained in the clearing reagent for making a biological material transparent is not particularly limited. Cyclodextrin is preferably contained at a concentration in a range of, for example, 1 mM or more to 5 mM or less. In addition, an amount by which the collagen binding compound is contained in the clearing reagent for making a biological material transparent is not particularly limited. The compound is preferably contained at a concentration in a range of, for example, 1 mM or more to 3 mM or less. In a case where cyclodextrins and/or a collagen binding compound is/are further contained, it is possible to greatly improve permeability of a reagent with respect to a biological material.

### (Solvent)

A "clearing reagent for making a biological material transparent" in accordance with an embodiment of the present invention is a solution containing a solvent in which urea is soluble. The solvent is not limited to any particular kind, provided that urea is soluble in the solvent. Preferably, water is used as a main solvent. Particularly preferably, only water is used as the solvent. Note that, in an embodiment of the present invention, what is meant by the expression "water is used as a main solvent" is that a volumetric percentage of water to all solvents used is larger than that of any other solvent, and preferably that water is used in an amount which accounts for more than 50% and 100% or less of a total volume of all solvents used. A "clearing reagent for making a biological material transparent" prepared by using water as a main solvent is referred to as a "clearing reagent for making a biological material transparent" as an aqueous solution.

In the case where water is used as a main solvent, the water can be mixed with dimethyl sulfoxide (DMSO) for application to a fixed sample, for example. It is expected that, for example, use of a mixture of DMSO and water to a fixed sample provides effects such as (i) improvement in permeability of the clearing reagent with respect to a biological material and (ii) facilitation of a clearing treatment with respect to a tissue having a keratin surface. An amount by which dimethyl sulfoxide is used is not particularly limited. Dimethyl sulfoxide is preferably in a range of 0.5 (v/v)% or more to 35.0 (v/v)% or less, more preferably in a range of 1.0 (v/v)% or more to 27.5 (v/v)% or less, and particularly preferably in a range of 1.5 (v/v)% or more to 25.0 (v/v)% or less, with respect to 100% by volume of the whole solvent.

Main advantages of the use of water as the solvent are as follows: 1) Urea and sorbitol, each of which is an active component of a "clearing reagent for making a biological material transparent" in accordance with an embodiment of the present invention, is excellent in solubility in water. Therefore, the use of water as the solvent makes it easily and inexpensive to preparer the clearing reagent for making a biological material transparent. 2) In comparison with a case where an organic solvent is used as a main solvent, the use of water as the solvent does not involve dehydration of a biological material when the biological material is subjected to a clearing treatment. Therefore, the use of water as the solvent can suppress unwanted shrinkage of a biological material. 3) In comparison with a case where an organic solvent is used as a main solvent, the use of water as the solvent markedly reduces the possibility of damaging a fluorescent protein. This makes it possible to observe, with use of a fluorescent protein, a biological material having been subjected to a clearing treatment. 4) The use of water as the solvent makes it possible to apply the clearing reagent in accordance with an embodiment of the present invention not only to a clearing treatment on a fixed material but also to a clearing treatment on a living material. 5) The use of water as the solvent makes a clearing treatment reversible as described later, that is, the use of water as the solvent can bring, as necessary, a biological sample having been subjected to a clearing treatment back to a state that it had before the clearing treatment. 6) In comparison with a case where an organic solvent is used as a main solvent, the use of water as the solvent enhances safety in handling of the clearing reagent.

A "clearing reagent for making a biological material transparent" in accordance with an embodiment of the present invention can be a buffer which can maintain a pH suitable for a biological material to be subjected to a clearing treatment. The pH of the "clearing reagent for making a biological material transparent" is not particularly limited. The pH is preferably in a range of 6.0 to 9.0, more preferably in a range of 6.5 to 8.5, and still more preferably in a range of 6.8 to 8.0. Further, a "clearing reagent for making a biological material transparent" in accordance with an embodiment of the present invention can have an osmotic pressure adjusted to a degree which further suppress deformation of a biological material to be subjected to a clearing treatment and which allows urea to sufficiently penetrate into the biological material.

Note that, as with the case of urea, the above solvent can also be used for the urea derivative.

### (Subject biological material)

A biological material to be subjected to a clearing treatment using a "clearing reagent for making a biological material transparent" in accordance with an embodiment of the present invention is not limited to any particular kind. Preferably, the biological material is a material derived from a plant or an animal, more preferably a material derived from an animal such as the one selected from fish, amphibians, reptiles, birds, and mammals, particularly preferably a material derived from a mammal. Examples of the mammal encompass, but are not particularly limited to: laboratory animals such as mice, rats, rabbits, guinea pigs, marmosets, and primates except for humans; pet animals such as dogs, cats, and ferrets; farm animals such as pigs, cows and horses; and humans.

Alternatively, the biological material can be an individual itself (except for a living human individual). Further alternatively, the biological material can be an organ, a tissue, or a cell taken from an individual of a multicellular organism. A "clearing reagent for making a biological material transparent" in accordance with an embodiment of the present invention has excellent ability to make a subject transparent. Therefore, even if the biological material is a tissue or an organ (e.g. the whole of or part of a brain) derived from a multicellular animal or an individual itself (e.g. embryo) of a multicellular animal which is not a human, the biological material can be subjected to a clearing treatment. The "clearing reagent for making a biological material transparent" in accordance with an embodiment of the present invention has an extremely large effect of suppressing deformation of a biological material, so as to be particularly suitable for making a fragile biological material transparent. Examples of the fragile biological material encompass plumule of plant cells, callus, an animal embryo at an early developmental stage, a stem cell, a primary cultured cell, a lump (e.g. spheroid, neurosphere, cell aggregates) which is obtained by three-dimensionally growing an established cell in a special culture environment.

Further, the biological material can be either of (i) a material fixed for a microscopic observation and (ii) a non-fixed material. In a case of using a fixed material, the material is preferably immersed in, e.g. a PBS solution or a 20(v/w)% sucrose-PBS solution adequately (e.g. for 24 hours or more) after being subjected to a fixing process. Furthermore, preferably, this material is embedded into an OCT compound and frozen by liquid nitrogen, thawed in PBS, and then fixed again by a 4 (v/w)% PFA (paraformaldehyde)-PBS solution.

Specific examples of the biological material encompass: biological tissue into which a fluorescent chemical substance injected; biological tissue stained with a fluorescent chemical substance; biological tissue into which a fluorescent protein-expressed cell is transplanted; and biological tissue taken from a genetically-modified animal in which a fluorescent protein is expressed.

### (Examples of particularly preferable composition of clearing reagent for making biological material transparent)

The following description will discuss examples of a particularly preferable composition of a "clearing reagent for making a biological material transparent", not all of which may fall within the scope of the present invention but are described only for illustrative purposes.

Clearing reagent (1) for making a biological material transparent:
An aqueous solution obtained by dissolving, in water, (i) urea at a concentration in a range of 1.5 M or more and 8.0 M or less and (ii) sorbitol at a concentration in a range of 15 (w/v)% or more to 50 (w/v)% or less. In a case where glycerol is further contained, glycerol is to be contained at a concentration preferably in a range of 2.5 (w/v)% or more to 35.0 (w/v)% or less, more preferably in a range of 3.0 (w/v)% or more to 30.0 (w/v)% or less, and still more preferably in a range of 5.0 (w/v)% or more to 15.0 (w/v)% or less. In a case where dimethyl sulfoxide is contained, dimethyl sulfoxide is to be contained preferably in a range of 0.5 (v/v)% or more to 35.0 (v/v)% or less, more preferably in a range of 1.0 (v/v)% or more to 27.5 (v/v)% or less, and particularly preferably in a range of 1.5 (v/v)% or more to 25.0 (v/v)% or less, with respect to 100% by volume of the whole solvent.

Clearing reagent (2) for making a biological material transparent:
An aqueous solution obtained by dissolving, in water, (i) urea at a concentration in a range of 3 M or more to 8 M or less, (ii) sorbitol at a concentration in a range of 15 (w/v)% or more to 50 (w/v)% or less, and further (iii) glycerol and/or dimethyl sulfoxide. Glycerol is to be contained at a concentration preferably in a range of 2.5 (w/v)% or more to 35.0 (w/v)% or less, more preferably in a range of 3.0 (w/v)% or more to 30.0 (w/v)% or less, and still more preferably in a range of 5.0 (w/v)% or more to 15.0 (w/v)% or less. In a case where dimethyl sulfoxide is contained, dimethyl sulfoxide is to be contained preferably in a range of 0.5 (v/v)% or more to 35.0 (v/v)% or less, more preferably in a range of 1.0 (v/v)% or more to 27.5 (v/v)% or less, and particularly preferably in a range of 1.5 (v/v)% or more to 25.0 (v/v)% or less, with respect to 100% by volume of the whole solvent.

Clearing reagent (3) for making a biological material transparent:
An aqueous solution obtained by dissolving, in water, (i) urea at a concentration in a range of 2 M or more to 4 M or less and (ii) sorbitol at a concentration in a range of 15 (w/v)% or more to 50 (w/v)% or less. In a case where glycerol is further contained, glycerol is to be contained at a concentration preferably in a range of 2.5 (w/v)% or more to 35.0 (w/v)% or less, more preferably in a range of 3.0 (w/v)% or more to 30.0 (w/v)% or less, and still more preferably in a range of 5.0 (w/v)% or more to 15.0 (w/v)% or less. In a case where dimethyl sulfoxide is contained, dimethyl sulfoxide is to be contained preferably in a range of 0.5 (v/v)% or more to 35.0 (v/v)% or less, more preferably in a range of 1.0 (v/v)% or more to 27.5 (v/v)% or less, and particularly preferably in a range of 1.5 (v/v)% or more to 25.0 (v/v)% or less, with respect to 100% by volume of the whole solvent.

Clearing reagent (4) for making a biological material transparent:
An aqueous solution obtained by dissolving, in water, (i) urea at a concentration in a range of 3 M or more to 5 M or less, (ii) sorbitol at a concentration in a range of 30 (w/v)% or more to 50 (w/v)% or less, and further (iii) glycerol and/or dimethyl sulfoxide. In a case where glycerol is contained, glycerol is to be contained at a concentration preferably in a range of 2.5 (w/v)% or more to 35.0 (w/v)% or less, more preferably in a range of 3.0 (w/v)% or more to 30.0 (w/v)% or less, and still more preferably in a range of 5.0 (w/v)% or more to 15.0 (w/v)% or less. In a case where dimethyl sulfoxide is contained, dimethyl sulfoxide is to be contained preferably in a range of 0.5 (v/v)% or more to 35.0 (v/v)% or less, more preferably in a range of 1.0 (v/v)% or more to 27.5 (v/v)% or less, and particularly preferably in a range of 1.5 (v/v)% or more to 25.0 (v/v)% or less, with respect to 100% by volume of the whole solvent.

Clearing reagent (5) for making a biological material transparent:
An aqueous solution obtained by dissolving, in water, (i) urea at a concentration in a range of 1.5 M or more and 4.0 M or less, (ii) sorbitol at a concentration in a range of 40 (w/v)% or more to 50 (w/v)% or less, and further (iii) glycerol and/or dimethyl sulfoxide. In a case where glycerol is contained, glycerol is to be contained at a concentration preferably in a range of 20 (w/v)% or more to 35 (w/v)% or less, and more preferably in a range of 22.5 (w/v)% or more to 30.0 (w/v)% or less. In a case where dimethyl sulfoxide is contained, dimethyl sulfoxide is to be contained preferably in a range of 0.5 (v/v)% or more to 35.0 (v/v)% or less, more preferably in a range of 1.0 (v/v)% or more to 27.5 (v/v)% or less, and particularly preferably in a range of 1.5 (v/v)% or more to 25.0 (v/v)% or less, with respect to 100% by volume of the whole solvent.

Clearing reagent (6) for making a biological material transparent:
An aqueous solution obtained by dissolving, in water, (i) urea at a concentration in a range of 5.0 M or more and 9.5 M or less and (ii) sorbitol at a concentration in a range of 15 (w/v)% or more to 40 (w/v)% or less. In a case where glycerol is contained, glycerol is to be contained at a concentration preferably in a range of 20 (w/v)% or more to 35 (w/v)% or less, and more preferably in a range of 22.5 (w/v)% or more to 30.0 (w/v)% or less. In a case where dimethyl sulfoxide is contained, dimethyl sulfoxide is to be contained preferably in a range of 0.5 (v/v)% or more to 35.0 (v/v)% or less, more preferably in a range of 1.0 (v/v)% or more to 27.5 (v/v)% or less, and particularly preferably in a range of 1.5 (v/v)% or more to 25.0 (v/v)% or less, with respect to 100% by volume of the whole solvent.

Clearing reagent (7) for making a biological material transparent:
An aqueous solution obtained by dissolving, in water, (i) urea at a concentration in a range of 3 M or more to 5 M or less and (ii) sorbitol at a concentration in a range of 35 (w/v)% or more to 45 (w/v)% or less. In a case where glycerol is contained, glycerol is to be contained at a concentration preferably in a range of 20 (w/v)% or more to 35 (w/v)% or less, and more preferably in a range of 22.5 (w/v)% or more to 30.0 (w/v)% or less. In a case where dimethyl sulfoxide is contained, dimethyl sulfoxide is to be contained preferably in a range of 0.5 (v/v)% or more to 35.0 (v/v)% or less, more preferably in a range of 1.0 (v/v)% or more to 27.5 (v/v)% or less, and particularly preferably in a range of 1.5 (v/v)% or more to 25.0 (v/v)% or less, with respect to 100% by volume of the whole solvent.

In any of the clearing reagents (1) through (7) for making a biological material transparent, a nonionic surfactant such as TritonX-100 is to be contained at a concentration preferably in a range of 0.025 (w/v)% or more to 5 (w/v)% or less, more preferably in a range of 0.05 (w/v)% or more to 0.5 (w/v)% or less, and particularly preferably in a range of 0.05 (w/v)% or more to 0.2 (w/v)% or less.

In a case where a transparency increasing component such as sucrose is contained in any of the clearing reagents (1) through (7) for making a biological material transparent, the transparency increasing component is to be contained at a concentration preferably in a range of 10.0 (w/v)% or more to 50.0 (w/v)% or less, and more preferably in a range of 20.0 (w/v)% or more to 40.0 (w/v)% or less.

In addition, any of the clearing reagents (1) through (7) for making a biological material transparent can further contain a compound which is selected from the group consisting of cyclodextrins and/or a collagen binding compound (N-acetyl-L-hydroxyproline and the like). Cyclodextrin is preferably contained at a concentration in a range of 1 mM or more to 5 mM or less. In addition, the collagen binding compound is preferably contained at a concentration in a range of 1 mM or more to 3 mM or less. A clearing reagent for making a biological material transparent, which contains a collagen binding compound and/or cyclodextrins, is particularly suitable for clearing biological materials containing white matters, and, among such biological materials, particularly suitable for clearing a spinal cord.

Note that in the case where the urea derivative (or a mixture of urea and the urea derivative) is used instead of urea, the clearing reagents (1) through (7) for making a biological material transparent can be prepared with the urea derivative (or the mixture) at the same concentration as the above-described concentration of urea.

### (Preparation of clearing reagent for making biological material transparent)

A "clearing reagent for making a biological material transparent" in accordance with an embodiment of the present invention can be prepared by dissolving, in a solvent, (i) "urea and/or a urea derivative" and "sorbitol" and (ii) "glycerol", "surfactant", "water-soluble macromolecular compound", "drying inhibition component", "transparency increasing component", and the like each of which is to be used as necessary. A procedure to dissolve or mix the above ingredients in/with a solvent is not particularly limited.

### [2. Example of method for clearing treatment using clearing reagent for making biological material transparent]

### (Clearing treatment step)

A method for a clearing a biological material with use of a "clearing reagent for making a biological material transparent" in accordance with an embodiment of the present invention includes a step (clearing treatment step) for causing the "clearing reagent" to permeate into the "biological material". More specifically, in this step, the "clearing reagent" permeates into the "biological material" in a container for a clearing treatment.

In the clearing treatment step, there is no particular limitation on the order in which the "clearing reagent" and the "biological material" are stored in the container for the clearing treatment. Preferably, the "clearing reagent" is stored first, and then the "biological material" is stored (i.e. the biological material is put into the clearing reagent).

A processing temperature at which the above clearing treatment step is performed is not particularly limited. Preferably, the processing temperature is in a range of 15°C or more to 45°C or less. A total processing time in which the clearing treatment is performed is not particularly limited. Preferably, the total processing time is in a range of 2 hours or more to 120 hours or less. A pressure at which the clearing treatment is performed is not particularly limited.

The clearing treatment step can be performed in stages with use of a plurality of kinds of clearing reagents for making a biological material transparent in accordance with an embodiment of the present invention. For example, 1) in a case where the clearing treatment step is performed in n stages (n is an integer of 2 or more), it is preferable to use clearing reagents so that sorbitol concentrations are increasingly higher as the number of stages progresses. This is because, in such a case, sorbitol efficiently permeates into a biological material.

Alternatively, 2) in a case where the clearing treatment step is performed in n stages (n is an integer of 3 or more), it is possible that (i) clearing reagents containing glycerol are used at least in the first stage and the nth stage and (ii) a clearing reagent(s) not containing glycerol is/are used at least once between the second stage and the (n-1)th stage. In this case, it is preferable that clearing reagents used in the respective stages between the first stage and the (n-1)th stage contain (i) sorbitol in an increased amount as the number of stages progresses and (ii) urea in an amount equal to or less than the amount in the previous stage. This is because, in such a case, expansion of a biological material by urea decreases gradually. Note, however, that it is preferable that the treatment in the nth stage is performed with use of a clearing reagent which (i) contains urea at a concentration in a range of 3 M or more to 5 M or less, (ii) contains sorbitol at a concentration in a range of 30 (w/v)% or more to 50 (w/v)% or less, and further (iii) contains glycerol and/or dimethyl sulfoxide.

Alternatively, 3) in a case where the clearing treatment step is performed in n stages (n is an integer of 2 or more), it is possible that (i) the treatment in the first stage is performed with use of a clearing reagent which (a) contains urea at a concentration in a range of 1.5 M or more and 4.0 M or less, (b) contains sorbitol in a range of 40 (w/v)% or more to 50 (w/v)% or less, and further (c) contains glycerol and/or dimethyl sulfoxide and (ii) the treatments in the subsequent stages are performed with use of clearing reagents each of which contains urea and sorbitol at concentrations higher and lower, respectively, than those of the clearing reagent used in the first stage. In this case, the biological material strongly shrinks in the first stage of treatment. Therefore, while a volume of the biological material is restored due to a decrease in shrinkage effect after the treatment in the first stage, the clearing reagents used in the subsequent stages can easily permeate into the biological material. Note that in a case where n is 3 or more, it is preferable that the treatment in the nth stage is performed with use of a clearing reagent which (i) contains urea at a concentration in a range of 3 M or more to 5 M or less, (ii) contains sorbitol at a concentration in a range of 30 (w/v)% or more to 50 (w/v)% or less, and further (iii) contains glycerol and/or dimethyl sulfoxide.

In each of the cases described above, it is preferable to add, between the adjacent stages of the clearing treatment step, a step of performing a treatment with PBS. This is because, in such a case, an original form of the biological material can be preserved.

The container for the clearing treatment which is used in the above clearing treatment step and in which the biological material having been subjected to the clearing treatment is stored can be preserved, e.g. at room temperature or in a low-temperature environment until the container is used in the below-described observation step. (clearing sample preserving step).

### (Pretreatment step)

As necessary, a biological material can be subjected, before a clearing treatment step, to a pretreatment with use of a pretreatment solution so that the clearing treatment can be performed smoothly. The pretreatment solution is preferably an aqueous solution which (i) contains sorbitol, (ii) contains glycerol and/or dimethyl sulfoxide, and (iii) contains neither urea nor the above-described urea derivative. Preferably, the pretreatment solution contains, for example, (i) a component which causes a reduction in cholesterol content of a biological material and/or (ii) a component which causes a reduction in collagen content of the biological material. The composition of the pretreatment solution will be described in [3. Kit for clearing treatment for making biological material transparent] below in detail.

A processing temperature at which the above pretreatment step is performed is not particularly limited. Preferably, the processing temperature is in a range of 15°C or more to 45°C or less. A total processing time in which the pretreatment is performed is not particularly limited. The total processing time is preferably in a range of 1 hour or more to 120 hours or less, and more preferably in a range of 2 hours or more to 48 hours or less. A pressure at which the pretreatment is performed is not particularly limited.

In a case where a cholesterol content of a biological material and/or a collagen content of the biological material is/are reduced through a pretreatment step, it is possible to greatly increase, in a clearing treatment step, permeability of a clearing reagent with respect to a biological material. This brings the effect of allowing a clearing reagent for making a biological material transparent to quickly permeate, for example, even a biological material which has not been subjected to (i) freezing with use of liquid nitrogen or the like and (ii) a thawing process at room temperature.

### (Step of observing biological material having been subjected to clearing treatment)

On the biological material having been subjected to the clearing treatment, an observation step with use of, for example, an optical microscope is then performed. On the biological material to be subjected to the observation step, a visualizing treatment step (e.g. staining or marking), as necessary, can be performed (i) before the clearing treatment step or (ii) after the clearing treatment step but before the observation step.

For example, in a case where the visualizing treatment step involves use of a fluorescent protein, a fluorescent protein gene is transferred into a living biological material before the clearing treatment step so that the fluorescent protein will be expressed therein.

In a case where the visualizing treatment step is (i) injection of a fluorescent chemical substance (which is not a fluorescent protein) into a biological material or (ii) staining of a biological material with a fluorescent chemical substance, the visualizing treatment step is preferably performed before the clearing treatment step. However, such the visualizing treatment step can be performed after the clearing treatment step. Alternatively, the visualizing treatment step can be staining of a biological material with a chemical substance which is not a fluorescent chemical substance.

The observation step can be performed with use of any type of optical microscope. For example, the observation step can be performed by employing a three-dimensional superresolution microscopy technique (e.g. STED, 3D PALM, FPALM, 3D STORM, or SIM). The observation step can be performed by employing a multi-photon excitation type (generally, two-photon excitation type) optical microscopy technique. However, since expansion of a biological material due to a clearing treatment is suppressed, it is possible to sufficiently observe the biological material with use of a one-photon excitation type optical microscopy technique.

Note that in an embodiment of the present invention, it is one of indications of a clearing (treatment) that a comparison made before and after a biological material is subjected to the clearing treatment shows that the biological material after the clearing treatment has increased light (particularly, visible light) transmittance.

### [2A. Other applications]

### (Step of removing component of clearing reagent for making biological material transparent)

A clearing treatment using a "clearing reagent for making a biological material transparent" in accordance with an embodiment of the present invention is reversible. As such, a biological material having been subjected to the clearing treatment can be brought back to a state that it had before the clearing treatment by, for example, immersing the biological material in an equilibrium salt solution so as to remove therefrom the components of the clearing reagent. Specific examples of the equilibrium salt solution encompass: equilibrium salt solutions (e.g. PBS and HBSS) which are buffered by phosphate; an equilibrium salt solution (TBS) which is buffered by tris hydrochloride; an artificial cerebrospinal fluid (ACSF); and basal media for cell culturing, such as MEM, DMEM, and Ham's F-12.

The use of the "clearing reagent for making a biological material transparent" does not cause denaturation or the like of a protein, etc. in the biological material (i) before and after the clearing treatment or (ii) in a case where, after the clearing treatment, the biological material is brought back to a state that it had before the clearing treatment. Accordingly, antigenicity of the protein, etc. in the biological material is preserved without a change. As such, for example, after a biological material is subjected to a clearing treatment and an optical microscopic observation, the biological material can be brought back to a state that it had before the clearing treatment, so as to undergo, for example, a detailed assay by a method of generally-used tissue staining or immunostaining.

In other words, another aspect of the present invention is a method for restoring a biological material which method includes a step of causing an equilibrium salt solution to permeate into a biological material having been made transparent by a clearing treatment by use of the "clearing reagent for making a biological material transparent", so that the biological material is brought back to a state that the biological material had before the clearing treatment.

### (Examples of visualizing treatment step for visualizing biological material to be subjected to observation step)

In a case where a biological material is subjected to a visualizing treatment by a method of immunostaining either during or after the biological material is subjected to a clearing treatment with use of a "clearing reagent for making a biological material transparent" in accordance with an embodiment of the present invention, it is preferable to employ, for example, the antibody composition and the immunization method disclosed in PCT International Publication, No. WO2014/010633A1. However, the present invention is not particularly limited to this antibody composition and this immunization method. The whole of the content disclosed in PCT International Publication, No. WO2014/010633A1 is incorporated as a part herein by reference.

Note that as disclosed in PCT International Publication, No. WO2014/010633A1, the antibody composition is a solution which contains (i) at least one compound selected from the group consisting of urea and a urea derivative, the at least one compound being contained at a concentration in a range of 0.1 M or more to less than 1.0 M and (ii) an antibody. The antibody composition preferably contains the at least one compound at a concentration in a range of 0.2 M or more and 0.5 M or less. The antibody composition preferably contains a surfactant, and more preferably contains a nonionic surfactant. In a case where a surfactant is contained, the surfactant is preferably contained at a concentration in a range of 0.025 (w/v)% or more to 0.2 (w/v)% or less.

An example of a schematic flow of immunostaining and an observation method using the antibody composition in accordance with PCT International Publication, No. WO2014/010633A1 is as follows. Note that any of the following steps can be performed under conditions similar to those of publicly known immunostaining methods.
Step 1): Step of preparing a sample (biological material) for immunostaining.
Step 2): Step of subjecting, as necessary, the sample thus prepared in the step 1) to an antigen activation treatment. The step 2) is performed by, for example, performing a treatment such as a heat treatment or a proteolytic treatment.
Step 3): Step of reducing background noise as necessary. The step 3) is performed by performing, for example, (i) an RNA degradative treatment for preventing contamination of unwanted RNA or (ii) a blocking treatment with use of a blocking treatment reagent such as serum or skimmed milk.
Step 4): Step of performing an antigen-antibody reaction between (i) the sample after the steps 1) through 3) and (ii) an antibody composition containing a primary antibody for immunostaining. Note that conditions of incubation in the step 4) can be decided according to, for example, antibody performance and sample size. For example, the incubation is performed by shaking for 6 hours to 5 days, preferably 2 days to 3 days. Preferably, a temperature for the incubation is, for example, approximately 4°C. A specific example of a concentration at which the primary antibody is contained in the antibody composition is a concentration in a range of 4 µg/mL or more to 40 µg/mL or less. Step 5): Step of washing the sample after the step 4). In the step 5), for example, the sample is rinsed with use of a solution containing, at a concentration in a range of 0.1 M or more to less than 1 M, at least one compound selected from the group consisting of urea and a urea derivative (urea derivative as consistently defined herein). An amount of the solution used is, for example, 9 ml to 15 ml for 0.3 g of the sample, preferably approximately 12 ml for 0.3 g of the sample. However, the present invention is not particularly limited to these amounts. The solution is preferably rinsed by shaking at room temperature for approximately 1 hour. However, the step 5) is not particularly limited to these temperature and time.
Step 6): Step of performing, as necessary, an antigen-antibody reaction between (i) an antibody composition containing a secondary or higher order antibody for immunostaining and (ii) the sample after the step 5). Note that conditions of incubation in the step 6) can be decided according to, for example, antibody performance and sample size. For example, the incubation is performed by shaking for 6 hours to 5 days, preferably 2 days to 3 days. Preferably, a temperature for the incubation is, for example, approximately 4°C. A specific example of a concentration at which the secondary or higher order antibody is contained in the antibody composition is a concentration in a range of 1 µg/mL or more to 10 µg/mL or less. Step 7): Step of washing the sample after the step 6). More specifically, the step 7) is performed as with the step 5).
Step 8): Step of visualizing, as necessary, results of the antigen-antibody reactions in the sample for immunostaining after the steps 1) through 7). For example, in a case where the primary antibody or the secondary or higher order antibody is labeled by an enzyme such as alkaline phosphatase, the visualization is performed by (i) reacting the sample with a substrate of the enzyme so as to generate a pigment and (ii) depositing the pigment. In a case where the primary antibody or the secondary or higher order antibody is labeled by a fluorescent dye such as fluorescein or rhodamine, observation in a step 9) below is performed directly while visualizing with a fluorescence microscope.
Step 9): Step of observing, with use of an optical microscope, the sample which has been immunostained through the steps 1) through 8).

In a case where the step 4) is performed and the step 6) is not performed in the above schematic flow, the antibody composition used in the step 4) is the antibody composition disclosed in PCT International Publication, No. WO2014/010633A1. In a case where the step 4) and the step 6) are both performed, (i) at least one of the antibody compositions used in the respective step 4) and the step 6) is the antibody composition disclosed in PCT International Publication, No. WO2014/010633A1 and (ii) preferably the antibody compositions used in the respective step 4) and the step 6) are each the antibody composition disclosed in PCT International Publication, No. WO2014/010633A1.

### [3. Kit for clearing treatment for making biological material transparent]

### (Kit for clearing treatment for making biological material transparent)

A "kit for a clearing treatment for making a biological material transparent" in accordance with an embodiment of the present invention includes at least one of the "clearing reagents for making a biological material transparent" described above. The kit for a clearing treatment for making a biological material transparent can include the plurality of kinds of clearing reagents for making a biological material transparent. In a case where a plurality of kinds of clearing reagents for making a biological material transparent are contained, for example, the plurality of kinds of clearing reagents can differ, from each other, in terms of (i) components contained and/or (ii) amounts of components contained. A combination of the clearing reagents satisfies, for example, at least one condition selected from the following 1) through 7). A representative example of the combination is that of the clearing reagents which satisfies the following condition 1).
1) Sorbitol contents vary depending on the individual clearing reagent.
2) Glycerol may or may not be contained, depending on the individual clearing reagent, and the amounts of glycerol, if contained at all, vary depending on the individual clearing reagent.
3) Urea contents or urea derivative contents vary depending on the individual clearing reagent.
4) A surfactant may or may not be contained, depending on the individual clearing reagent, and the amounts of surfactant, if contained at all, vary depending on the individual clearing reagent. 5) Dimethyl sulfoxide (DMSO) may or may not be contained, depending on the individual clearing reagent, and the amounts of dimethyl sulfoxide, if contained at all, vary depending on the individual clearing reagent.
6) Cyclodextrins may or may not be contained, depending on the individual clearing reagent, and the amounts of cyclodextrins, if contained at all, vary depending on the individual clearing reagent.
7) A collagen binding compound, which is selected from the group consisting of N-acetyl-L-hydroxyproline and the like, may or may not be contained, and the amounts of compound, if contained at all, vary depending on the individual clearing reagent.

A "kit for a clearing treatment for making a biological material transparent" is preferably a kit in which at least one selected from the group consisting of the following clearing reagents A) and B) is included: A) a clearing reagent for making a biological material transparent, including: at least one compound selected from the group consisting of urea and a urea derivative, the at least one compound being contained at a concentration in a range of 3 M or more to 5 M or less; and sorbitol at a concentration in a range of 30 (w/v)% or more to 50 (w/v)% or less and B) a clearing reagent for making a biological material transparent, including: at least one compound selected from the group consisting of urea and a urea derivative, the at least one compound being contained at a concentration in a range of 5 M or more and 9.5 M or less; and sorbitol at a concentration in a range of 20 (w/v)% or more to 40 (w/v)% or less, and more preferably a kit in which at least one selected from the group consisting of the following clearing reagents A1) and B) is included: A1) a clearing reagent for making a biological material transparent, including: at least one compound selected from the group consisting of urea and a urea derivative, the at least one compound being contained at a concentration in a range of 3 M or more to 5 M or less; and sorbitol at a concentration in a range of 35 (w/v)% or more to 45 (w/v)% or less and B) a clearing reagent for making a biological material transparent, including: at least one compound selected from the group consisting of urea and a urea derivative, the at least one compound being contained at a concentration in a range of 5 M or more and 9.5 M or less; and sorbitol at a concentration in a range of 20 (w/v)% or more to 40 (w/v)% or less.

The "kit for a clearing treatment for making a biological material transparent" can further include at least one selected from: a "container for a clearing treatment" for use in the clearing treatment step; a "biological material holding tool (e.g. tweezers)"; an "equilibrium salt solution" and a "pretreatment solution" each for bringing a biological material after a clearing treatment back to a state that it had before the clearing treatment; an "antibody for immunostaining" for use in a visualizing treatment step of visualizing the biological material; a "fluorescent chemical substance" for use in the visualizing treatment step of visualizing the biological material; and an "instruction manual for the kit". Note that, the instruction manual for the kit explains, for example, a procedure for the clearing treatment method as described in the [2. Example of method for clearing treatment using clearing reagent for making biological material transparent] section above.

The pretreatment solution is a liquid for treating a biological material before a clearing treatment so as to smoothly perform the clearing treatment. The pretreatment solution is preferably an aqueous solution which (i) contains no urea, (ii) contains sorbitol, and further (iii) contains glycerol and/or dimethyl sulfoxide. More preferably, the pretreatment solution contains, for example, (i) a component which causes a reduction in cholesterol content of a biological material and/or (ii) a component which causes a reduction in collagen content of the biological material. Examples of the component which causes a reduction in cholesterol content encompass cyclodextrins. More specifically examples of the component encompass α-cyclodextrin, a derivative of α-cyclodextrin, β-cyclodextrin, a derivative of β-cyclodextrin (such as methyl-p-cyclodextrin), γ-cyclodextrin, and a derivative of γ-cyclodextrin. An amount by which cyclodextrins are contained in the pretreatment solution is not particularly limited. Cyclodextrins are preferably contained at a concentration in a range of 1 mM or more to 5 mM or less. Examples of the component which causes a reduction in collagen content encompass at least one compound selected from the group consisting of N-acetyl-L-hydroxyproline and the like. The collagen binding compound is preferably contained at a concentration in a range of 1 mM or more to 3 mM or less.

The pretreatment solution can contain not only the component which causes a reduction in cholesterol content of a biological material and/or the component which causes a reduction in collagen content of the biological material, but also, as necessary, the components contained in a clearing reagent in accordance with an embodiment of the present invention for making a biological material transparent. In such a case, components contained in the clearing reagent can be contained in the pretreatment solution by the same amounts. Among the components, preferably sorbitol is contained in the pretreatment solution. In such a case, sorbitol is to be contained at a concentration preferably in a range of 15 (w/v)% or more to 50 (w/v)% or less, more preferably in a range of 15 (w/v)% or more to 25 (w/v)% or less, and still more preferably in a range of 17 (w/v)% or more to 23 (w/v)% or less. Glycerol is also a component preferably contained in the pretreatment solution. In such a case, glycerol is to be contained at a concentration preferably in a range of 2.5 (w/v)% or more to 35.0 (w/v)% or less, more preferably in a range of 2.5 (w/v)% or more to 15.0 (w/v)% or less, and particularly preferably in a range of 2.5 (w/v)% or more to 7.5 (w/v)% or less. In a case where dimethyl sulfoxide is contained, dimethyl sulfoxide is to be contained at a concentration preferably in a range of 0.5 (v/v)% or more to 35.0 (v/v)% or less, more preferably in a range of 1.0 (v/v)% or more to 27.5 (v/v)% or less, and particularly preferably in a range of 1.5 (v/v)% or more to 25.0 (v/v)% or less, with respect to 100% by volume of the whole solvent in the pretreatment solution. Note that as is the case of the clearing reagent for making a biological material transparent in accordance with an embodiment of the present invention, the solvent in the pretreatment solution preferably contains water as a main solvent.

### [Examples]

The following description will discuss an embodiment of the present invention in more detail with reference to Examples, Comparative Example, and the like below. Note, however, that the present invention is not limited to these.

### [Method of experiment]

### =ScaleS solutions=

The following reagents were purchased for preparation of ScaleS solutions: urea crystals (Wako pure chemical industries), Triton X-100 (Wako pure chemical industries), D(-)-sorbitol (Wako pure chemical industries), methyl-β-cyclodextrin (Tokyo Chemicals), γ-cyclodextrin (Wako pure chemical industries), N-acetyl-L-hydroxyproline (Kyowa Hakko Bio), dimethyl sulfoxide (DMSO) (Wako pure chemical industries), glycerol (Sigma), and Triton X-100 (Nacalai Tesque). The reagents were dissolved in or added to water (see Fig. 8), and stirred until well mixed (heating by a microwave oven was carried out for preparation of ScaleS4, ScaleS4(0), ScaleSQ(0), and ScaleSQ(5)). A stock solution of phosphate-buffered saline (10x PBS(-)) was used for making ScaleS0. The final concentrations of the ingredients contained in the solution were adjusted by diluting the resulting mixed solutions with water. Note that ScaleS4(0) is a solution having a composition identical to that of ScaleS4 except that ScaleS4 (0) contains no Triton X-100. In Examples and Comparative Example, the unit of sorbitol concentration is (w/v)%, the unit of glycerol concentration is (w/v)%, the unit of sucrose concentration is (w/v)%, the unit of N-acetyl-L-hydroxyproline is (w/v)%, the unit of DMSO concentration is (v/v)%, and the unit of Triton X-100 concentration is (w/v)%, unless specified otherwise.

### =Clearing treatment with use of ScaleS solution=

The most suitable protocol at present for making a hemisphere of a mouse transparent is as follows.

First, permeability of a sample was increased by incubating, for 12 hours, the sample in a ScaleS0 solution containing 20% sorbitol, 5% glycerol, 1 mM methyl-β-cyclodextrin, 1 mM γ-cyclodextrin, 1% N-acetyl-L-hydroxyproline, and 3% DMSO (first step).

Then, the sample after the first step was sequentially incubated in ScaleS1, ScaleS2, and ScaleS3 (second step).

Then, the sample after the second step was washed by PBS for 6 hours to replace the sample by PBS, so that a state of the sample before the treatment was restored (deScale treatment: third step).

Then, the sample after the third step was incubated in ScaleS4 for 12 hours as a step before observation. ScaleS4 was used also as a mounting medium.

In the protocol above, the temperatures for the incubation were each 37°C, and only the temperature for washing by PBS was 4°C. The incubation was carried out basically in an orbital oscillator (70 rpm/min. to 80 rpm/min.) in which a temperature can be controlled.

### =Antibodies for AbScale=

The following antibodies were used. Alexa488-6E10, which is mouse mAb to amyloid-β conjugated with Alexa Fluor 488, was purchased from Covance (SIG-39347). Cy5-A60 was made by treating, with use of Cy5 bis-reactive dye (GE Healthcare, PA25500), mouse mAb to NeuN (A60) (Millipore, MAB377). Cy3-aNeuN, which is rabbit polyAb to NeuN conjugated with Cy3, was purchased from Millipore (ABN78C3). Rabbit polyAb to Iba1 and goat polyAb to rabbit IgG conjugated with Alexa Fluor 546 were purchased from Wako pure chemical industries and Molecular Probes, respectively.

These antibodies were each mixed with an antibody treatment solution (AbScale solution: see Fig. 10) so that an antibody concentration would be 5 µg/mL, and were then used for AbScale treatment. Note that the antibody treatment solution is an aqueous solution having a composition including 0.33 M urea, 0.1%(w/v) Triton X-100, and x1 time PBS. A rinse solution (AbScale rinse solution: see Fig. 10) is an aqueous solution having a composition including 2.5% BSA, 0.05% (wt/vol) Tween-20, and x 0.1 times PBS.

### =Sample preparation (mouse)=

Adult mice and aged mice (8 weeks old to 80 weeks old) were sufficiently anesthetized with pentobarbital (Somnopentyl) and transcardially perfused with 4% PFA/PBS(-). The whole brains were taken out and subjected to post-fixation in 4% PFA/PBS(-) at 4°C for 10 hours or 3 days. Then, the hemispheres obtained from the whole brain and slices containing the hippocampus were made transparent (cleared) by ScaleS directly or made transparent (cleared) after being subjected to AbScale treatment or ChemScale treatment. The experimental procedures and housing conditions for animals were approved by the institute's animal experiments committee, and the experimental procedures were carried out as approved.

### =Sample preparation (human)=

Frozen postmortem brain (frontal cortex-temporal lobe) blocks of Alzheimer's disease patients (60 years old to 80 years old) (provided by Philadelphia University) were thawed and fixed in 4% PFA/PBS(-) at 4°C for 8 hours. A 3 mm x 4 mm cube was excised from each block, and was subjected to AbScale treatment with use of Alexa488-6E10 (at a dilution ratio of 1:200), Cy5-A60 (at a dilution ratio of 1:200), and rabbit anti-lbal polyAb (at a dilution ratio of 1:200). The sample was further reacted with goat anti-rabbit IgG polyAb conjugated with Alexa Fluor 546 (at a dilution ratio of 1:500).

### =Image segmentation=

The image data of brain samples having been subjected to the AbScale treatment were acquired by the SPIM system (Lightsheet Z.1 and ZEN software, ZEISS), and stored in TIFF format. To extract target objects, namely, microglia (Alexa546-lba1 signals) and amyloid-β plaques (Alexa488-6E10 signals), on a massive scale, a semi-automated image segmentation method was developed. The method began with the manual assignment of objects. Each cell or plaque was fully masked by hand. Binary masking data were produced with use of ImageJ software (64 bit, version 10.7). Then, the target picture was automatically segmented according to the Otsu method. Because of multilevel signals and variation of the background level, the method is extremely helpful in selecting the most suitable threshold for picture segmentation in each masked region. All processes involving the image segmentation were performed with use of a custom-made program written in C₊₊ language and the OpenCV Library. Finally, the picture was sharpened by applying a coarse median filter (radius 2.0).

### =Measurement of distances=

Commercial software Volocity version 6.3 (PerkinElmer) was applied to the binary masking data sets to measure the distances from the center of each microglial cell to the nearest amyloid-β plaque (both surface and center). The number of microglia that were located within 50 µm from the amyloid-β plaque surface was counted and plotted by a custom-made program written in C₊₊ language together with commercial software (Igor Pro version 6.3.4.1).

### =Image re-slicing=

To create various two-dimensional stacked images from an entire three-dimensional dataset, the inventors of the present invention developed a re-slicing program. This program integrates xy image planes of all numbers systematically. This provides an array of z-stacked images having different thicknesses. This program can also be used to re-slice the three-dimensional reconstruction data to create x-stacked images or y-stacked images. This program was written in C₊₊ language.

### [Results of experiment]

### (1) Combination of sorbitol with urea

The inventors of the present invention initially screened compounds included in the group consisting of sugars and sugar alcohols, including erythritol, fructose, glycerol, mannitol, sorbitol, sucrose, and xylitol, for the ones that render fixed brain samples transparent in combination with urea. It was found that sorbitol cleared the mouse cerebral cortex most effectively in combination with urea.

By combining sorbitol with urea, it is possible to make a sample transparent while an original volume of the sample is preserved. The reasons for this phenomenon are not necessarily clear. However, one possible reason is deemed as follows: although both urea and sorbitol have tissue-clearing capability, urea causes hydration (tissue expansion), whereas sorbitol causes dehydration (tissue shrinkage). In hopes of inducing dehydration of tissues, glycerol in addition to sorbitol was employed. Whereas sorbitol is hydrophilic, glycerol is amphipathic. Therefore, by addition of glycerol, action in lipophilic areas inside the brain was further increased.

The inventors of the present invention found that the addition of sorbitol is critical for the overall performance, and thus named the urea-based clearing solutions containing sorbitol "ScaleS". The inventors of the present invention attempted to devise, by combining sorbitolcontaining solutions, ScaleS protocol that is intended for adult (aged) mammalian brains. At present, the most suitable protocol features incubation of a fixed brain sample in a plurality of solutions from ScaleS0 based on PBS through ScaleS4 (b of Fig. 9). The formulas of all the ScaleS solutions are shown in Fig. 8. After the sample was equilibrated, the sample became substantially transparent in ScaleS4 (a of Fig. 1), and the volume of the sample converged to nearly 100% of the original ((A) of Fig. 12). In comparison with the case where a treatment was carried out with use of ScaleA2 (Hama et al., Nat Neuroscience 14 (11), 1481-8 (2011)) of a conventional clearing reagent for making a biological material transparent, the effect of preserving an original form of the biological material was remarkable ((A) and (B) of Fig. 12). The step of treating ScaleA2 is schematically shown in a of Fig. 9.

### (2) Great capabilities of ScaleS to preserve signal and structure

CUBIC is a recently devised method that uses mixtures of urea, amyl alcohols, and Triton X-100. The method involves an incubation of a sample in a reagent (CUBIC Reagent 1) containing 15% Triton X-100 for approximately 7 days. Because of the high concentration of Triton X-100, CUBIC features powerful clearing capability and therefore enables rapid whole-brain imaging by single-photon excitation microscopy, such as light-sheet microscopy (LSFM) or specific plane illumination microscopy (SPIM) after simple treatment of the sample. For the same reason, however, CUBIC may not ensure preservation of signal and structure.

In the present example, in contrast, the ScaleS method limits Triton X-100 concentration to low concentrations (such as less than 0.2%) throughout the entire procedure (Fig. 8). It is expected that such low Triton X-100 concentrations would not pose any deleterious effects on biological structures. Therefore, the Scale technology will allow for the visualization of specific fine structures, such as synapses, at both light and electron microscopy scales after global 3D reconstruction. This technology will also ensure adequate preservation of FP (fluorescent protein) signals. The inventors of the present invention examined the preserving and clearing capabilities of ScaleS. The fixed brain of a YFP-H mouse (20 weeks old) was split into two hemispheres. The left hemisphere was cleared by ScaleS, and the right hemisphere was cleared by PBS. The two hemispheres were comparatively imaged for transmission and YFP fluorescence (b of Fig. 1). The ScaleS-treated hemisphere became substantially transparent, and showed stronger YFP fluorescence than did the PBS-treated hemisphere; the fluorescence observation evidently benefitted from the transparency. A similar result was obtained with use of hemispheres prepared from ChR2-YFP mice (c of Fig. 1). In contrast, CUBIC-treated samples showed weaker YFP fluorescence than did PBS-treated samples (Fig. 13). This fluorescence reduction by CUBIC was partly due to the quenching of YFP by CUBIC Reagent 1, as was revealed by quenching experiments in which recombinant YFP was used. Because CUBIC Reagent 1 is highly alkaline (pH of 11.3), irreversible denaturation of YFP occurs during the treatment. It should be noted, however, that the fluorescence reduction is also caused by the leakage of YFP. This is because CUBIC Reagent 1 contains 15% Triton X-100, which is expected to induce the leakage of YFP from a sample.

The inventors of the present invention previously demonstrated the reversibility of ScaleA2 treatment for retrospective 2D-IHC. Such reversibility was examined also for ScaleS and CUBIC (Fig. 14). Cytoskeleton proteins, such as MAP2 and GFAP, were sufficiently immunolocalized in the sections obtained from ScaleS-treated samples and CUBIC-treated samples. However, substantial differences in synaptic proteins were noted between the ScaleS-treated samples and CUBIC-treated samples. Whereas ScaleS treatment preserved the immunostaining of presynaptic and postsynaptic proteins and immunostaining of marker molecules existing on a surface of an immature neuronal cell membrane, CUBIC treatment attenuated the intensity and/or specificity of the immunostaining.

Furthermore, synaptic ultrastructures were comparatively examined by electron microscopy. After fixation with 4% PFA, a hemisphere was cleared by ScaleS treatment. Then, the hemisphere was replaced by PBS, so that components of ScaleS were removed (deScaling). This restored a state of the sample before the clearing treatment (restoration). Then, a 1-mm cube was prepared from the sample which has been restored to the state before the clearing treatment, and was used to prepare ultrathin sections for transmission electron microscopy (TEM) observation. The observation provided a relatively wide view covering adjacent somata of two layer V pyramidal neurons (d of Fig. 1). Membrane integrity was so well preserved that the subcellular organelles in one of the neurons (on the left) could be surveyed from the nucleus to the cell surface extensions, such as the dendrite (Den) and axon hillock (AH). It is therefore possible to examine the ultrastructures in the context of neuronal subcellular organization. When zooming in on the axon initial segment (AIS), for example, an electron-dense region was discovered on the cell surface (e of Fig. 1). Further magnification (f of Fig. 1) revealed that the region reflected a symmetric synapse with pleomorphic vesicles present in the axon terminal (AT). Its location and ultrastructure suggest that this synapse was an inhibitory and axo-axonic synapse. In contrast, asymmetric (presumably excitatory) synapses with transparent and round vesicles inside AT are often identified in the neuropil region (g and h of Fig. 1). Likewise, ultrathin sections from CUBIC samples were prepared. As was anticipated from the high concentration (15%) of Triton X-100 contained in CUBIC Reagent 1, ultrathin sections, in which various fine structures, such as mitochondria, dendrite, and myelin sheath, other than plasma membranes of nerve cells, were markedly disrupted, were observed in the TEM images (Figs. 15 and 16).

### (3) Quantitative three dimensional (immuno) histochemistry

The inventors of the present invention previously discovered that in a case where a sample having been cleared with use of a clearing treatment reagent containing urea as a main component is replaced by PBS so that components such as urea is removed from the sample (deScaled), the deScaled samples are permeable to macromolecules such as antibodies. To use this feature, the inventors of the present invention have already devised a method called AbScale, which fully immunostains structures inside several-mm-thick brain samples (Fig. 10).

Then, the inventors of the present invention applied AbScale to brains from aged mouse models of Alzheimer's disease (AD). 6E10, which is a mouse monoclonal antibody (mAb) that reacts with amino acids 1-16 of Aβ, was used for characterizing the spatial distribution of Aβ plaques. This antibody has been used in molecularly targeted therapy. For example, intracranial injection of 6E10 was devised to lower brain Aβ. In this experiment, a commercially available fluorescent 6E10 (Alexa488-6E10) was used. However, because 6E10 reacts with some precursors of Aβ, such as APP and CTF-β, it is possible that Alexa488-6E10 signals are found inside neurons. The inventors of the present invention therefore used a new AD mouse model (AppNL-F) that overproduces Aβ42. To examine the position of the Alexa488-6E10 signals relative to neuronal nuclei, the inventors of the present invention attempted the combined use of mouse anti-NeuN mAb (A60) conjugated with Cy5 (A60-Cy5).

In an AbScale solution, a fixed hemisphere (6 mm x 4 mm x 4 mm) obtained from a 20-month-old AppNL-F/NL-F mouse was reacted with both Alexa488-6E10 and Cy5-A60 (pre-cut staining). After treatment with 20% sucrose solution, the hemisphere was embedded in OCT compound and cut in the coronal plane into 50-µm-thick sections (a of Fig. 2). A section containing the hippocampus was mounted on a glass slide and reacted with rabbit anti-NeuN polyclonal Ab and then goat anti-rabbit IgG Ab conjugated with Cy3 (post-cut staining). Then, three images for Cy3 (b of Fig. 2), Cy5 (c of Fig. 2), and Alexa488 (d of Fig. 2) fluorescence were acquired with a stereomicroscope (a of Fig. 17: corresponding to Fig. 2). First, Cy3-αNeuN (post-cut staining) and Cy5-A60 images were compared to evaluate the diffusivity of Cy5-A60 inside the whole hemisphere. The Cy5 signal was substantially strong irrespective of the depth from the hemisphere surface, and matched the Cy3 signal completely (e of Fig. 2). This result indicates the efficient penetration of Cy5-A60 mAb into the hemisphere, and demonstrates the validity of AbScale using Cy5-A60. Then, the Cy5-A60 image was compared with the other pre-cut staining image of Alexa488-6E10 (f of Fig. 2). The Alexa488-labeled Aβ plaques were localized to regions deficient in Cy5-labeled neuronal nuclei (g and h of Fig. 2). This suggests that the Aβ plaques resided extracellularly. The Alexa488-labeled Aβ plaques were found mostly in the cortex and to a lesser extent in the thalamus (f of Fig. 2); the same pattern of Aβ distribution had been observed by 2D-IHC in coronal sections obtained from a 20-month-old AppNL-F/NL-F mouse. It was therefore concluded that the penetration of Alexa488 was sufficient to fluorescently label all Aβ plaques residing inside the brain samples each having a size of 4 mm or less.

### (4) ChemScale and AbScale for visualizing Aβ amyloid

While developing the original Scale method, the inventors of the present invention found that fixed brain samples became considerably permeable to fluorescent substances during or after clearing treatment with use of ScaleA2 and/or ScaleB4. In a previous work, the inventors of the present invention successfully achieved nuclear counterstaining of a ScaleA2-treated brain block with 4',6-diamidino-2-phenylindole (DAPI). This method, called ChemScale (Fig. 11), can be applied to large samples in general.

The inventors of the present invention combined ChemScale with AbScale with an eye to achieving multi-color imaging. The inventors of the present invention attempted to monitor Aβ plaques by use of Alexa488-6E10 and PP-BTA-1 which is a benzothiazole derivative that shows strong affinity for Aβ aggregates, and whose color could be distinguished from that of Alexa488-6E10 (b of Fig. 17: corresponding to a of Fig. 3 and b of Fig. 3). This combined approach was applied to the hemisphere of an 18-month-old AppNL-F/NL-F mouse. The inventors of the present invention first obtained a sweeping view of fluorescently-labeled Aβ plaques that were scattered throughout the entire cortex (a of Fig. 3). PP-BTA-1 highlighted amyloid deposition in cerebral vasculature, a condition termed cerebral amyloid angiopathy, more strongly than Alexa488-6E10. The inventors of the present invention then zoomed in on individual Aβ plaques. Detailed 3D reconstruction of a representative fluorescent deposit revealed a round and hollow morphology (a of Fig. 3, inset) which reflects the shape of previously reported Alexa488-6E10-stained Aβ plaques. In contrast, the intense fluorescence of PP-BTA-1 was seen in the core of the Aβ plaques. These results indicate that the staining and imaging depth using Alexa488-6E10 and PP-BTA-1 was sufficient for visualizing Aβ amyloid covering the whole hemisphere. Then, the hemisphere of a 9-month-old AppNL-F/NL-F mouse was stained and imaged in the same manner, but only sparse labeling was noted (b of Fig. 3).

As a subtype of ChemScale, the inventors of the present invention previously labeled mouse blood vessels with red fluorescence for the 3D reconstruction of vasculature. In the same way, the inventors of the present invention transcardially perfused an anesthetized AppNL-F/NL-F mouse (20 months old) with Texas-Red-labeled lectin. After subsequent fixation, the left hemisphere was AbScaled with Alexa488-6E10 and imaged with the SPIM system (c of Fig. 17: corresponding to c of Fig. 3). 3D reconstruction in a quadratic prism located in the cerebral cortex showed that all Aβ plaques were either in direct contact with or localized near blood vessels (c of Fig. 3).

### (5) Microglia-Aβ plaque association in aged mouse and human brains

Many studies of 2D sections have indicated that microglial cells are localized in increasing numbers around Aβ plaques in Alzheimer's disease (AD) patients and models. However, the inventors of the present invention are of the opinion that the imaging of discrete cell populations and the quantitative measurement of their genetic properties must be performed in 3D space.

To acquire a comprehensive perspective of how microglial activation is associated with amy-loidosis, the inventors of the present invention applied a dual-color AbScale method to a 2-mm-thick coronal brain slice obtained from a 20-month-old AppNL-F/NL-F mouse (a of Fig. 4). The slice was subjected to reaction with Alexa488-6E10 and rabbit anti-lbal pAb plus anti-rabbit IgG Ab conjugated with Alexa546 in order to immunostain Aβ plaques and microglia, and then optically cleared. A region containing the somatosensory cortex and hippocampal CA1 (cuboid in a of Fig. 4) was able to be viewed across the 2-mm-thick slice with use of the SPIM system (d of Fig. 17: corresponding to Fig. 4). In addition to 6E10-positive plaques, Iba1-positive microglia with typical sizes and shapes were clearly visualized. Their 3D reconstructions were extended inside the observed region, and two renderings (xy and xz planes) were created (b and c of Fig. 4, respectively). The latter view shows fluorescence distribution along the z-axis, and therefore reflects the penetration efficiency of the Abs. Because there was no signal attenuation in the deepest region, the inventors of the present invention concluded that the Abs penetrated from both surfaces efficiently. Then, the inventors of the present invention selected a cortical region containing 37 well-isolated Aβ plaques (d of Fig. 4). The inventors of the present invention found 120 microglia that were localized near (closer than a distance of 60 µm) the Aβ plaques. After image segmentation, the inventors of the present invention determined the size and shape of individual plaques and the locations (barycenters) of both plaques and microglia. The inventors of the present invention used home-made algorithm to automatically measure the distance of each microglial center to the nearest plaque edge in 3D space (e of Fig. 5). Because resting and activated microglia have ramified and amoeboid shapes, respectively, and therefore make it easy to characterize their morphology in 3D space, the inventors of the present invention were able to judge the activation/resting state of each microglial cell segmented. In addition to their proximity to the plaques, the morphological features of the activated microglia may provide information about the severity of plaque neuritis. For instance, the inventors of the present invention discovered two neighboring plaques having a substantially identical size (f of Fig. 4) but different spatial association with microglia (g of Fig. 4). One plaque was in direct contact with or accompanied by nine active microglia. This suggests its acute neuritic state (h of Fig. 4, #2). The other plaque was not closely associated with any microglia, and its far neighbors were nearly all resting (h of Fig. 4, #1); this plaque was therefore suggested to be obsolete. The histograms of the two Aβ plaques show the distribution of the distances of the activated and resting microglia to the plaque edge (h of Fig. 4, bottom). For all the 37 plaques, their neighboring 120 microglia were analyzed by the same method (i of Fig. 4). The distances of both activated and resting microglia to the plaques were relatively long; while 102 microglia were localized far (further than a distance of 21 µm) from the plaque edges, only 18 cells were associated with the plaques extremely closely (closer than a distance of 21 µm). The histograms of individual plaques are shown in a of Fig. 18. The inventors of the present invention defined, as obsolete plaques, plaques that were not associated by any activated microglia in their vicinity (closer than a distance of 21 µm). 25 plaques out of 37 (68%) were classified "obsolete".

The same analyses were performed by use of a slice prepared from a 10-month-old AppNL-F/NL-F mouse (j of Fig. 4); 107 microglia were found in the vicinity of 27 plaques in a plaque-rich region (k of Fig. 4). The histogram of the 27 plaques showed that their distances to the nearest plaques were widely distributed (I of Fig. 4). The histograms of individual plaques are likewise presented (b of Fig. 18). According to the above-mentioned definition, interestingly, only 2 out of 27 (7.4%) plaques were classified "obsolete". Although much less Aβ plaques were found at this early stage, a larger fraction of the plaques appeared to be in the acute neuritic state than at 20 months.

Then, the inventors of the present invention also 3D visualized the microglia-plaque association in postmortem brain samples from Alzheimer's Disease patients likewise by use of the dual-color AbScale approach. Importantly, to discriminate extracellular Aβ plaques from intracellular Alexa488-6E10 signals, Cy5-A60 was additionally used to identify neuronal nuclei. The 3D reconstructions classified 9 patients' brain blocks into 2 groups; 3 samples were endowed with dense cored plaques (Fig. 19, left: cored plaques (+)) and 6 others were not (Fig. 19, right: cored plaques (-)). In one sample (#1617) of the former group, for example, 11 cored plaques were clearly observed, but clustering of Iba1-positive microglia was not observed for such abundant plaque region (a of Fig. 5). Further examination of the rendering revealed that most of the cored plaques were isolated from microglia (b of Fig. 5), and that very few showed microglial association (c of Fig. 5). In a sample (#1523) of the latter group, in contrast, no clear cored plaques were observed, but substantial microglial clustering was observed in the 3D rendering (d of Fig. 5). During the examination of serial xy images, it was found that each cluster of microglia was closely associated with a "diffuse plaque". This type of plaque was vague in comparison with the surrounding intracellular Aβ accumulation, and could not be defined in the volumetric ray casting image data (d of Fig. 5). To discover such hidden structures that might span several xy images, a custom-made program was used to construct a full set of z-stacked images each containing 2 xy images to 12 xy images (e of Fig. 5). Diffuse plaques became evident in several of the z-stacked images (f and g of Fig. 5). Then the cuboid regions were used to examine whether or not the diffuse plaques were associated with microglia (h and i of Fig. 5, respectively). Inside the block sample (#1523), total of 26 diffuse plaques were identified, among which 22 were found to interact with microglia. Such microglial association of diffuse plaques was often observed in other patients' samples (Fig. 19). Optical slicing was indeed effective for threshold optimization for the segmentation of obscure images. Because a re-slicing approach does not involve mechanical sectioning, the re-slicing approach makes it possible to identify structures of different sizes. Interestingly, diffuse plaques were hardly observed in the cored plaque-containing samples (Fig. 19, left). This supports the view that diffuse plaques are the precursors of cored plaques.

### (6) ScaleSQ: quick versions of Scale applicable to brain slices

While it is not easy to clear whole aged mouse brains quickly, the inventors of the present invention have realized that a considerable number of researchers are interested in using brain slices rather than whole brains. In particular, those who are currently engaged in stere-ology will sufficiently benefit from transparent brain slices (1 mm to 2 mm). Then, the question is whether or not it is possible to devise a quick clearing methods specific to brain slices.

Another intriguing question was to what extent it would be possible to increase urea concentration in the presence of sorbitol that counteracts the hydration effect of urea. The inventors of the present invention found that a solution containing 9.1 M urea and 22.5% sorbitol had the power to quickly clear few-mm-thick brain slices of adult mice. Remarkably, the solution did not contain Triton X-100, and was named ScaleSQ(0). A 1-mm-thick brain sample of a 8-week-old YFP-H mouse was apparently cleared after incubation in ScaleSQ(0) at 37°C for 1 hour to 2 hours (a through c of Fig. 6). The inventors of the present invention compared YFP fluorescence between the cleared half (d of Fig. 6) and the control half (Fig. 20) to verify that this approach fully preserved the YFP fluorescence. The cleared half was incubated in ScaleS4(0) (Fig. 8) for 2 hours, and then used in a quick 3D imaging experiment that employed the SPIM system. The 3D reconstruction of YFP-expressing neurons (e of Fig. 6) in a part of the cortex (white box in d of Fig. 6) was completed within 1 minute. ScaleSQ(0) solution is expected to preserve ultrastructures very well. In fact, the inventors of the present invention evaluated the membrane integrity in a restored sample by performing EM observation (f of Fig. 6).

Despite the apparent transparency achieved with ScaleSQ(0) (a through c of Fig. 6), SPIM imaging revealed opacity remaining inside the slice specimen. To increase the transparency of the cleared slice, Triton X-100 was added at various concentrations to ScaleSQ(0). The inventors of the present invention determined that the upper limit of the Triton X-100 concentration that sufficiently allows FP fluorescence to be preserved was 5%. ScaleSQ(5) (Fig. 8) allowed for the clearing of a 1-mm-thick slice without any loss of YFP fluorescence (g through I of Fig. 6). The cleared sample was highly suitable for the SPIM imaging of YFP-expressing neurons (k of Fig. 6). However, preservability of ultrastructures was unfavorable due to the effect of Triton X-100 concentration (I of Fig. 6).

### (7) ScaleSS: ScaleS having further increased transparency

In order to evaluate a clearing reagent for making a biological material transparent (ScaleSS) whose transparency (light-transmitting property) is further increased than a ScaleS4 solution, ScaleSS20 (composition: 4 M urea, 40% sorbitol, 20% sucrose, 20% DMSO, and 0.2% Triton X-100) and ScaleSS40 (composition: 3.56 M urea, 35.56% sorbitol, 35.56% sucrose, 17.78% DMSO, and 0.18% Triton X-100) were prepared. To verify an increase in transparency by use of ScaleSS20 and ScaleSS40, the following was performed: a 17-week-old YFP-H mouse as an object of a clearing treatment was sufficiently anesthetized with use of pentobarbital (Somnopentyl), and then transcardially perfused with use of 4% PFA/PBS (-). Then, the whole brain was taken out and subjected to post-fixation in 4% PFA/PBS (-) at 4°C for 3 days, so that a fixed whole brain was prepared. The whole brain was then subjected to the first step through the third step described in "=Clearing treatment with use of ScaleS solution=" in [Method of experiment] above. Then, the sample after the third step as a step before observation was incubated in ScaleS4 for 189 days. On the day following 189 days of incubation, the whole brain was split into a right hemisphere and a left hemisphere. The right hemisphere was incubated in ScaleSS20 or ScaleSS40 for 1 day, and then subjected to a clearing treatment was performed. On the other hand, the left hemisphere was incubated in ScaleS4 again for 1 day for comparison of transparency.

The hemispheres, which have been subjected to the clearing treatment with use of ScaleSS20, ScaleSS40, or ScaleS4, were compared (Fig. 21) in terms of permeability (Permeability in Fig. 21), signal intensity of YFP fluorescence (Fluorescence) in Fig. 21), and change of volume (Expansion (Area) in Fig. 21). The results revealed that permeability and fluorescence signal intensity are increased by a treatment with ScaleSS20 or ScaleSS40, whereas, as is the case of ScaleS4, there is no effect on the change of volume.

Then, to evaluate transparency in a case where a whole brain is treated with ScaleSS40, the following was performed: The whole brain of a YFP-H mouse (17 weeks old) was fixed with 4% PFA, and subjected to the first step through the third step described above. Then, the sample after the third step was incubated in ScaleS4 for 59 days as a step before observation, so that the whole brain thus incubated was prepared. The whole brain sample was observed while the whole brain sample was taken out of ScaleS4 (in air) and immersed in ScaleS4 (in liquid) (indicated as S4D25 in the upper portion of Fig. 22). Then, the whole brain sample was further incubated in ScaleSS40 for 2 days, and was observed while the whole brain sample was taken out of ScaleSS40 (in air) and immersed in ScaleSS40 (in liquid). The results confirmed that incubation in ScaleSS40 for 2 days increased permeability. (Fig. 22). The results thus revealed that the composition of ScaleSS further increased a light-transmitting property.

### <Comparative Example 1>

A transgenic mouse having a fluorescent protein YFP expressed in its neurons of the nervous system (YFP-H line: provided by Professor Joshua Sanes of Harvard University, U.S.A. [reference] Feng et al. Neuron, 28: 41-51, 2000) was used to perform perfusion fixation of the mouse and to take out and fix a biological material (cerebrum). The method of the fixation is as follows. At room temperature, a peristaltic pump or a syringe was used to perfuse a fixing solution (4 (w/v)% paraformaldehyde/PBS, pH of 7.5 to 8.0) from the left ventricle of the heart, so that the animal was systemically fixed. Then, the cranial bones were removed from the animal thus systemically fixed, and the organ (e.g. whole of the brain) was carefully taken out as a biological material. Then, the organ thus taken out was immersed in an ice-cold fixing solution (4 (w/v)% paraformaldehyde/PBS, pH of 7.5 to 8.0) at 4°C for 8 hours to 12 hours.

Note that after being taken out, the cerebrum was split into the left hemisphere and the right hemisphere, which were then subjected to fixation.

Then, one of the two cerebral hemispheres thus obtained was immersed, at room temperature for 4 days, in a clearing reagent for making a biological material transparent having a composition shown below. The other one of the cerebral hemispheres was immersed, at room temperature for 4 days, in a comparative reagent having a composition shown below. The results are shown in Fig. 7. Note that in Fig. 7, the cerebral hemisphere on the left is the one treated with the comparative reagent, and the cerebral hemisphere on the right is the one treated with the clearing reagent for making a biological material transparent. With the comparative reagent, the cerebral hemisphere was substantially not cleared.

The comparison thus made revealed that a combination of urea and sorbitol increases transparency.

Composition of clearing reagent for making a biological material transparent:
Aqueous solution in which 40%(w/v) sorbitol, 2 M urea, 10%(w/v) glycerol, and 0.2% (w/v) TritonX-100 are contained in water.

Composition of comparative reagent:
Aqueous solution in which 40%(w/v) erythritol, 2 M urea, 10%(w/v) glycerol, and 0.2% (w/v) TritonX-100 are contained in water.

The present invention is not limited to the embodiments or examples described above, but can be altered in many ways by a person skilled in the art within the scope of the Claims. An embodiment derived from a proper combination of technical means disclosed in different embodiments is also encompassed in the technical scope of the present invention.

### Industrial Applicability

The present invention can provide a (i) clearing reagent to make a biological material transparent for preparation of a biological material for use in optical observation and (ii) use of the clearing reagent.

## Claims

1. A clearing reagent for making a biological material transparent, comprising:
- at least one compound selected from the group consisting of urea and a urea derivative;
- sorbitol; and
- a surfactant,
**characterized in that** the clearing reagent contains
(i) the at least one compound at a concentration in a range of 3 M or more to 5 M or less; and the sorbitol at a concentration in a range of 30 (w/v)% or more to 50 (w/v)% or less, or
(ii) the at least one compound at a concentration in a range of 5 M or more to 9.5 M or less; and the sorbitol at a concentration in a range of 20 (w/v)% or more to 40 (w/v)% or less, and
the surfactant is contained at a concentration in a range of more than 0 (w/v)% to 5 (w/v)% or less.

2. The clearing reagent as set forth in claim 1, wherein the clearing reagent is a solution containing urea as the compound.

3. The clearing reagent as set forth in claim 1 or 2, wherein the surfactant is a nonionic surfactant.

4. The clearing reagent as set forth in any one of claims 1 through 3, further comprising glycerol.

5. The clearing reagent as set forth in any one of claims 1 through 4, further comprising Cyclodextrins.

6. The clearing reagent as set forth in any one of claims 1 through 5, wherein the biological material is (i) a tissue or an organ derived from a multicellular animal or (ii) a multicellular animal which is not a human.

7. A system for a clearing treatment for making a biological material transparent, comprising:
- a clearing reagent recited in claim 1; and
- a biological material which has been isolated, the clearing reagent having permeated into the biological material so that the biological material is made transparent

8. The system as set forth in claim 7, wherein the surfactant is a nonionic surfactant, and the clearing reagent further contains glycerol and cyclodextrins.

9. A method for making a biological material transparent, comprising the step o causing a clearing reagent recited in claim 1 to permeate into a biological material which has been isolated, so that the biological material is made transparent.

10. The method as set forth in claim 9, wherein the surfactant is a nonionic surfactant, and the clearing reagent further contains glycerol and cyclodextrins.

11. A kit for a clearing treatment for making a biological material transparent, comprising a clearing reagent recited in any one of claims 1 through 6.

12. The kit as set forth in claim 11, wherein a plurality of kinds of clearing reagents for making a biological material transparent which differ in sorbitol concentration are included.

13. The kit as set forth in claim 12, further comprising as a pretreatment solution for use in a clearing step, an aqueous solution which contains a) sorbitol and b) glycerol and/or dimethyl sulfoxide and which contains no urea.

## Patentansprüche

1. Klärungsreagens, um ein biologisches Material transparent zu machen, umfassend:
- mindestens eine Verbindung, die aus der Gruppe, die aus Harnstoff und Harnstoffderivat besteht, ausgewählt ist;
- Sorbit; und
- ein Tensid,
**dadurch gekennzeichnet, dass** das Klärungsreagens Folgendes umfasst
(i) die mindestens eine Verbindung in einer Konzentration in einen Bereich von 3 M oder mehr bis 5 M oder weniger; und den Sorbit in einer Konzentration in einem Bereich von 30 (w/v)% oder mehr bis 50 (w/v)% oder weniger, oder
(ii) die mindestens eine Verbindung in einer Konzentration in einem Bereich von 5 M oder mehr bis 9,5 M oder weniger; und den Sorbit in einer Konzentration in einem Bereich von 20 (w/v)% oder mehr bis 40 (w/v)% oder weniger, und
das Tensid in einer Konzentration in einem Bereich von mehr als 0 (w/v)% bis 5 (w/v)% oder weniger umfasst ist.

2. Klärungsreagens nach Anspruch 1, wobei das Klärungsreagens eine Lösung ist, die Harnstoff als die Verbindung umfasst.

3. Klärungsreagens nach Anspruch 1 oder 2, wobei das Tensid ein nichtionisches Tensid ist.

4. Klärungsreagens nach einem der Ansprüche 1 bis 3, ferner umfassend Glycerin.

5. Klärungsreagens nach einem der Ansprüche 1 bis 4, ferner umfassend Cyclodextrine.

6. Klärungsreagens nach einem der Ansprüche 1 bis 5, wobei das biologische Material (i) ein Gewebe oder ein Organ aus einem Vielzeller oder (ii) ein Vielzeller, der kein Mensch ist, ist.

7. System für eine Klärungsbehandlung, um das biologische Material transparent zu machen, umfassend:
- das Klärungsreagens nach Anspruch 1; und
- das biologische Material, das isoliert wurde, wobei das Klärungsreagens in das biologische Material eingedrungen ist, sodass das biologische Material transparent wird.

8. System nach Anspruch 7, wobei das Tensid ein nichtionisches Tensid ist, und das Klärungsreagens ferner Glycerin und Cyclodextrine umfasst.

9. Verfahren, um das biologische Material transparent zu machen, umfassend den Schritt des Veranlassens, dass das Klärungsreagens nach Anspruch 1 in das biologische Material, das isoliert wurde, eindringt, sodass das biologische Material transparent wird.

10. Verfahren nach Anspruch 9, wobei das Tensid ein nichtionisches Tensid ist, und das Klärungsreagens ferner Glycerin und Cyclodextrine umfasst.

11. Kit zur Klärungsbehandlung, um das biologische Material transparent zu machen, umfassend das Klärungsreagens nach einem der Ansprüche 1 bis 6.

12. Kit nach Anspruch 11, wobei mehrere Arten von Klärungsreagenzien, um das biologische Material transparent zu machen, die sich durch ihre Sorbitkonzentration unterscheiden, enthalten sind.

13. Kit nach Anspruch 12, ferner umfassend eine wässrige Lösung als Vorbehandlungslösung zur Verwendung in einem Klärungsschritt, die a) Sorbit und b) Glycerin und/oder Dimethylsulfoxid und keinen Harnstoff umfasst.

## Revendications

1. Réactif de compensation permettant de rendre transparent un matériau biologique, comprenant :
- au moins un composé choisi dans le groupe constitué de l'urée et d'un dérivé d'urée ;
- du sorbitol ; et
- un tensioactif,
**caractérisé en ce que** le réactif de compensation contient
(i) l'au moins un composé à une concentration dans une plage comprise entre 3 M ou plus et 5 M ou moins ; et le sorbitol à une concentration dans une plage comprise entre 30 % (p/v) ou plus et 50 % (p/v) ou moins, ou
(ii) l'au moins un composé à une concentration dans une plage comprise entre 5 M ou plus et 9,5 M ou moins ; et le sorbitol à une concentration dans une plage comprise entre 20 % (p/v) ou plus et 40 % (p/v) ou moins, et
le tensioactif est contenu à une concentration dans une plage comprise entre plus de 0 % (p/v) et 5 % (p/v) ou moins.

2. Réactif de compensation selon la revendication 1, dans lequel le réactif de compensation est une solution contenant de l'urée en tant que composé.

3. Réactif de compensation selon la revendication 1 ou la revendication 2, dans lequel le tensioactif est un tensioactif non ionique.

4. Réactif de compensation selon l'une quelconque des revendications 1 à 3, comprenant en outre du glycérol.

5. Réactif de compensation selon l'une quelconque des revendications 1 à 4, comprenant en outre des cyclodextrines.

6. Réactif de compensation selon l'une quelconque des revendications 1 à 5, dans lequel le matériau biologique est (i) un tissu ou un organe dérivé d'un animal multicellulaire ou (ii) un animal multicellulaire qui n'est pas un humain.

7. Système pour un traitement de compensation permettant de rendre transparent un matériau biologique, comprenant :
- un agent de compensation indiqué dans la revendication 1 ; et
- un matériau biologique qui a été isolé, l'agent de compensation ayant pénétré dans le matériau biologique de telle sorte que le matériau biologique est rendu transparent.

8. Système selon la revendication 7, dans lequel le tensioactif est un tensioactif non ionique, et le réactif de compensation contient en outre du glycérol et des cyclodextrines.

9. Procédé permettant de rendre transparent un matériau biologique, comprenant l'étape permettant d'amener un réactif de compensation indiqué dans la revendication 1 à pénétrer dans un matériau biologique qui a été isolé, de telle sorte que le matériau biologique est rendu transparent.

10. Procédé selon la revendication 9, dans lequel le tensioactif est un tensioactif non ionique, et le réactif de compensation contient en outre du glycérol et des cyclodextrines.

11. Kit destiné à un traitement de compensation permettant de rendre transparent un matériau biologique, comprenant un réactif de compensation indiqué dans l'une quelconque des revendications 1 à 6.

12. Kit selon la revendication, dans lequel une pluralité de types de réactifs de compensation permettant de rendre transparent un matériau biologique qui diffère en termes de concentration de sorbitol sont inclus.

13. Selon la revendication 12, comprenant en tant que solution de prétraitement destinée à être utilisée dans une étape de compensation, une solution aqueuse qui contient a) du sorbitol et b) du glycérol et/ou du diméthylsulfoxyde et qui ne contient pas d'urée.
